(19) Europäisches Patentamt / European Patent Office / Office européen des brevets

(11) **EP 3 288 457 B1**

(12) **EUROPEAN PATENT SPECIFICATION**

(45) Date of publication and mention
of the grant of the patent:
**01.09.2021 Bulletin 2021/35**

(21) Application number: **16728371.2**

(22) Date of filing: **27.04.2016**

(51) Int Cl.:
*A61B 5/1455* (2006.01)     *A61B 5/00* (2006.01)
*A61B 5/0245* (2006.01)

(86) International application number:
**PCT/IB2016/052379**

(87) International publication number:
**WO 2016/178119 (10.11.2016 Gazette 2016/45)**

(54) **POSITIONING A MEDICAL DEVICE BASED ON OXYGEN SATURATION MEASUREMENTS**

POSITIONIERUNG EINER MEDIZINISCHEN VORRICHTUNG AUF BASIS VON
SAUERSTOFFSÄTTIGUNGSMESSUNGEN

POSITIONNEMENT D'UN DISPOSITIF MÉDICAL REPOSANT SUR DES MESURES DE
SATURATION EN OXYGÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.04.2015 US 201514696523
27.04.2015 US 201514696513**

(43) Date of publication of application:
**07.03.2018 Bulletin 2018/10**

(73) Proprietor: **LifeWatch Technologies Ltd.
7670202 Rehovot (IL)**

(72) Inventors:
• **LEVANT, Anna
7621017 Rehovot (IL)**
• **BRACHIA HALEVI, Zohar
5226714 Ramat Gan (IL)**
• **AMIRIM, Mordehay
7624806 Rehovot (IL)**

(74) Representative: **Philips Intellectual Property &
Standards
High Tech Campus 5
5656 AE Eindhoven (NL)**

(56) References cited:
**WO-A1-98/43071          WO-A1-2013/036718
WO-A2-2008/120154        US-A- 5 273 036
US-A1- 2004 138 538      US-A1- 2004 215 244
US-A1- 2005 283 082      US-A1- 2013 030 259**

**EP 3 288 457 B1**

**Description**

**BACKGROUND OF THE INVENTION**

Oxygen saturation measurements provide highly valuable information about the state of a user. Results of oxygen saturation measurements depend upon the location of measurement and may be required to be taken over relatively long periods of time.

For example, US 2013/030259 A1 discloses a monitoring system suitable for attachment to a surface of a subject and for monitoring physiological signals of a subject wearing the system, and methods using such a monitoring system. The monitoring system comprises at least one first sensor, which can receive a first signal, and at least one second sensor, which can receive a second physiological signal from said subject. The sensors are controlled by a microelectronic system, wherein monitoring data based on the second physiological signal is under control of the monitoring data based on the first signal. The physiological signal or the monitoring data based on the first signal is one or more selected from heart rate, blood pressure, blood pH, respiration, skin and/or core body temperature, snoring sound or other sounds of the subject, electromyography, submental EMG, galvanic skin response, electrocardiography, electroencephalography, phonocardiogram, arterial oxygen saturation, muscle activity, motion, emotions, arterial saturation of carbon monoxide, sensors for physiological gases, such as a gas exhaled from the lungs, such as exhaled nitrogen oxide.

WO 2008/120154 A2 relates to a precordial device comprising a sternal notch alignment portion; and at least one measurement device connected to the precordial device, wherein when the sternal notch alignment portion is aligned with a sternal notch of a patient, the precordial device is in alignment with the patients' chest such that a consistent result can be obtained by the at least one measurement device.

US 2004/215244 A1 describes an external defibrillator for delivering a defibrillation waveform to a patient, which includes chest electrodes for detecting at least one ECG signal on the patient and for delivering the defibrillation waveform to the patient, a defibrillation circuit for shaping and delivering the defibrillation waveform, a pulse sensor for detecting the presence of a cardiac pulse in the patient, wherein the pulse sensor has a pulse signal as its output, and memory and processing circuits for analyzing the pulse signal in conjunction with the ECG signal to determine whether the patient has a pulse.

WO 98/43071 A1 discloses a method of measuring a blood constituent value using data comprising a single data set, which comprises: (a) determining a plurality of possible blood constituent values using a plurality of blood constituent value calculators, each of the blood constituent value calculators using the single data set, each of the possible blood constituent values having a confidence level associated therewith based on at least one quality metric; and (b) arbitrating between the plurality of possible blood constituent values with regard to the confidence levels to determine a measure of the blood constituent.

US 2004/138538 A1 relates to methods and devices for reducing noise effects in a system for measuring a physiological parameter, comprising receiving an input signal; obtaining an assessment of the signal quality of the input signal; selecting coefficients for a digital filter using the assessment of signal quality; and filtering the input signal using the digital filter, without comparing the filter's output signal with the input signal.

WO 2013/036718 A1 describes a system and method for evaluating physiological signals to determine whether or not a physiological signal corresponds to a medically relevant condition. In this context, a system and method is disclosed for determining acceptability of physiological signals that involves monitoring a subject with one or more channels of a physiological data capture or monitoring device or devices, such as an electrocardiogram, a pulse oximeter and/or a respiration trace, and acquiring signals from such device or devices.

There is a growing need to provide methods for accurate oxygen saturation measurements that can be easily taken over long periods of time.

**SUMMARY OF THE INVENTION**

The present invention provides a method for measuring oxygen saturation of a user, the method including: receiving, by a computerized device, first and second detection signals and electrocardiograph signals; wherein the first detection signals result from an illumination, by an oxygen saturation sensor included in a device that is removably attached to a user, of a sternal angle of a user by infrared pulses; wherein the second detection signals result from an illumination, by the oxygen saturation sensor, of the sternal angle of a user by visible light pulses; wherein the electrocardiograph signals are detected by an electrocardiography sensor that is included in the device; generating a first waveform template that may be responsive to the first detection signals; generating a second waveform template that may be responsive to the second detection signals; calculating an indication of the oxygen saturation characteristic of the user in response to the first and second detection signals; detecting cardiac cycle durations that are based upon the first and second detection signals; detecting electrocardiography based cardiac cycle durations; and evaluating a quality of the indication of the oxygen saturation characteristic of the user in response to the first waveform template, the second waveform template, the cardiac cycle's durations and the electrocardiography based cardiac cycle durations.

The method may include applying a high-pass filter and a bilateral filter on the first detection signals to provide first filtered detection signals.

The generating of the first waveform template may include filtering the first detection signals to provide first filtered detection signals; and detecting first cardiac cycle waveforms in the first filtered detection signals.

The detecting of the cardiac cycle durations may include measuring durations of the first cardiac cycle waveforms.

The generating of the first waveform template may include converting the first cardiac cycle waveforms to first duration-normalized cardiac cycle waveforms that have a same duration.

The generating of the first waveform template may be responsive to at least some of the first duration-normalized cardiac cycle waveforms.

The generating of the first waveform template equals an average of at least some of the first duration-normalized cardiac cycle waveforms.

The generating of the first waveform template further may include calculating, for each first duration-normalized cardiac cycle waveform, a similarity score indicative of a similarity between the first duration-normalized cardiac cycle waveform and other first duration-normalized cardiac cycle waveforms.

The generating of the first waveform template further may include ignoring at least one first duration-normalized cardiac cycle waveform based upon similarity scores of the first duration-normalized cardiac cycle waveforms.

The calculating, for each first duration-normalized cardiac cycle waveform, of the similarity score may include calculating a plurality of Pearson correlation coefficients between the first duration-normalized cardiac cycle waveform and a plurality of other first duration-normalized cardiac cycle waveforms.

The calculating, for each first duration-normalized cardiac cycle waveform, of the similarity score may include summing the plurality of Pearson correlation coefficients.

The method may include calculating qualities of at least some of the first cardiac cycle waveforms; and wherein the quality of the first and second detection signals may be responsive to the qualities of the at least some of the first cardiac cycle waveform.

The calculating of the qualities of at least some of the first cardiac cycle waveforms may include comparing the at least some of the first cardiac cycle waveforms to the first waveform template.

The calculating of the qualities of at least some of the first cardiac cycle waveforms may include calculating correlations between shapes of the at least some of the first cardiac cycle waveforms and a shape of the first waveform template.

The calculating of the qualities of at least some of the first cardiac cycle waveforms may include converting at least some of the first cardiac cycle waveforms to first duration-normalized and peak-normalized cardiac cycle waveforms and calculating relationships between shapes of the first duration-normalized and peak-normalized cardiac cycle waveforms and a shape of the first waveform template; and wherein the first duration-normalized and peak-normalized cardiac cycle waveforms may be a same duration and a same peak value as the first waveform template.

The calculating of the qualities of at least some of the first cardiac cycle waveforms may include calculating relationships between peaks of the at least some of the first cardiac cycle waveforms and a peak of the first waveform template.

The calculating of the qualities of at least some of the first cardiac cycle waveforms may include calculating relationships between durations of the at least some of the first cardiac cycle waveforms and durations of corresponding electrocardiography based cardiac cycle durations.

A non- transitory computer readable medium is described herein, that stores instructions that once executed by a computerized device cause the computerized device to execute the steps of: receiving first and second detection signals and electrocardiograph signals; wherein the first detection signals result from an illumination, by an oxygen saturation sensor included in a device that may be removably attached to a user, of a sternal angle of a user by infrared pulses; wherein the second detection signals result from an illumination, by the oxygen saturation sensor, of the sternal angle of a user by visible light pulses; wherein the electrocardiograph signals may be detected by an electrocardiography sensor that may be included in the device; generating a first waveform template that may be responsive to the first detection signals; generating a second waveform template that may be responsive to the second detection signals; calculating an indication of the oxygen saturation characteristic of the user in response to the first and second detection signals; detecting cardiac cycle durations that may be based upon the first and second detection signals; detecting electrocardiography based cardiac cycle durations; and evaluating a quality of the indication of the oxygen saturation characteristic of the user in response to the first waveform template, the second waveform template, the cardiac cycle's durations and the electrocardiography based cardiac cycle durations.

A medical device is also described herein, that includes a processor that includes one or more hardware components. The processor may be configured to (i) receive first and second detection signals and electrocardiograph signals; wherein the first detection signals result from an illumination, by an oxygen saturation sensor included in a device that may be removably attached to a user, of a sternal angle of a user by infrared pulses; wherein the second detection signals result from an illumination, by the oxygen saturation sensor, of the sternal angle of a user by visible light pulses; wherein the electrocardiograph signals may be detected by an electrocardiography sensor that may be included in the device; (ii) generate a first waveform template that may be responsive to the first detection signals; (iii) generate a second waveform template that may be responsive to the second detection signals; (iv) calculate an indication of the oxygen saturation characteristic of the user in response to the first and second detection signals; (v) detect cardiac cycle durations that may be based upon the first and second detection signals; (vi) detect electrocardiography based cardiac cycle durations; and (vii) evaluate a quality of the indication of the oxygen saturation characteristic of the user in response to the first waveform template, the second waveform template, the cardiac cycle's durations and the electrocardiography based cardiac cycle durations.

Also described herein is a method that may include receiving, by a computerized device, first detection signals generated as a result of an illumination, by infrared pulses, of a current portion of a sternum of a user; receiving, by the computerized device, second detection signals generated as a result of an illumination, by visible light pulses, of the current portion of the sternum of the user; and evaluating, by the computerized device, a quality of the first and second detection signals; and determining whether the current portion of the sternum of the user may be a sternal angle of the user; wherein the determining may be responsive to the quality of the first and second detection signals. The computerized device may be a server, a laptop computer, a desktop computer, a mobile phone, a personal data assistant, a medical monitor or any type of computerized system that has one or more hardware component.

The method may include illuminating the current portion of the sternum of the user by the infrared pulses and by the visible light pulses.

The illuminating may be executed by an oxygen saturation sensor that belongs to the computerized device.

The receiving of the first and second detection signals may include receiving the first and second detection signals from a device that differs from the computerized device.

The method may include determining that the current portion of the sternum of the user may be the sternal angle of the user when the quality of the first and second detection signals exceeds a predetermined quality threshold.

The evaluating of the quality of the first and second detection signals may include generating a first waveform template in response to the first detection signals.

The evaluating of the quality of the first and second detection signals may include detecting first cardiac cycle waveforms and generating a first waveform template in response to the first cardiac cycle waveforms.

The generating of the first waveform template may be followed by determining relationships between one or more first cardiac cycle waveform and the first waveform template.

The generating of the first waveform template may include : filtering the first detection signals to provide first filtered detection signals; and detecting first cardiac cycle waveforms in the first filtered detection signals.

The generating of the first waveform template may include converting the first cardiac cycle waveforms to first duration-normalized cardiac cycle waveforms that have a same duration.

The converting may be followed by calculating, for each first duration-normalized cardiac cycle waveform, a similarity score that may be indicative of a similarity between the first duration-normalized cardiac cycle waveform and other first duration-normalized cardiac cycle waveforms.

The method may include calculating, for each first duration-normalized cardiac cycle waveform, the similarity score by calculating a plurality of Pearson correlation coefficients between the first duration-normalized cardiac cycle waveform and a plurality of other first duration-normalized cardiac cycle waveforms.

The calculating a plurality of Pearson correlation coefficients may be followed by applying a first mathematical function on the plurality of Pearson correlation coefficients to provide the similarity score of the first duration-normalized cardiac cycle waveform.

The generating of the first waveform template may include ignoring at least one first duration- normalized cardiac cycle waveform based upon similarity scores of the first duration-normalized cardiac cycle waveforms to provide relevant first duration- normalized cardiac cycle waveforms.

The generating of the first waveform template may be responsive to the relevant first duration-normalized cardiac cycle waveforms.

The method may include calculating qualities of at least some of the first cardiac cycle waveforms; and wherein the quality of the first and second detection signals may be responsive to the qualities of at least some of the first cardiac cycle waveforms.

The calculating of a quality of a first cardiac cycle waveform out of the at least some of the first cardiac cycle waveforms may include comparing the first cardiac cycle waveform to the first waveform template.

The calculating of a quality of a first cardiac cycle waveform out of the at least some of the first cardiac cycle waveforms may include comparing calculating a correlation between a shape of the first cardiac cycle waveform and a shape of the first waveform template.

The calculating of a quality of a first cardiac cycle waveform out of the at least some of the first cardiac cycle waveforms may include converting the first cardiac cycle waveform to a first duration-normalized and peak-normalized cardiac cycle waveform and calculating a relationship between a shape of the first duration-normalized and peak- normalized cardiac cycle waveform and a shape of the first waveform template.

The calculating of a quality of a first cardiac cycle waveform out of the at least some of the first cardiac cycle waveforms may include comparing a relationship between a peak of the first cardiac cycle waveform and a peak of the first waveform template.

The method wherein a calculating of a quality of a first cardiac cycle waveform out of the at least some of the first cardiac cycle waveforms may include calculating a relationship between a peak of the first cardiac cycle waveform and a peak of the first waveform template.

Also described herein is a non- transitory computer readable medium that stores instructions that once executed by a computerized device cause the computerized device to execute the steps of: receiving, by a computerized device, first detection signals generated as a result of an illumination, by infrared pulses, of a first portion of a sternum of a user; receiving, by the computerized device, second detection signals generated as a result of an illumination, by visible light pulses, of the first portion of the sternum of the user; evaluating, by the computerized device, a quality of the first and second detection signals; and determining whether the first portion of the sternum of the user may be a sternal angle of the user; wherein the determining may be responsive to the quality of the first and second detection signals.

Also described herein is a device that may be removably attached to a user and may include an oxygen saturation sensor, wherein the oxygen saturation sensor may be configured to: generate first detection signals responsive to an illumination, by infrared pulses, of a first portion of a sternum of a user; generate second detection signals responsive to an illumination, by visible light pulses, of the first portion of the sternum of a user; and evaluate a quality of the first and second detection signals; and determine whether the first portion of the sternum of the user may be the sternal angle of the user, in response to the quality of the first and second detection signals.

## BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof may best be understood by reference to the following detailed description when read with the accompanying drawings in which:

FIG. 1 illustrates the sternum and the ribs of a person;

FIG. 2 is an exploded view of a device useful in an embodiment of the method of the invention;

FIG. 3 illustrates a placement of the device of FIG. 2 on a chest of a user according to an embodiment of the invention;

FIG. 4 illustrates a placement of the device of FIG. 2 on a chest of a user according to an embodiment of the invention;

FIG. 5 is a schematic diagram of various components of the device of FIG. 2;

FIG. 6 is a timing diagram according to an embodiment of the invention;

FIG. 7 illustrates a method according to an embodiment of the invention;

FIG. 8 illustrates a method according to an embodiment of the invention;

FIG. 9 illustrates a method according to an embodiment of the invention;

FIG. 10 illustrates a device that is removably attached to a person useful in an embodiment of the method of the invention;

FIG. 11 illustrates a method for positioning the device according to an embodiment of the invention;

FIG. 12 illustrates a method according to an embodiment of the invention;

FIGs. 13-15 illustrate a stage of processing the first and second detection signals to evaluate a quality of the first and second detection signals according to an embodiment of the invention;

FIG. 16 illustrates first detection signals and first filtered detection signals according to an embodiment of the invention;

FIG. 17 illustrates first detection signals, first filtered detection signals, first cardiac cycle waveforms, first waveform template, first duration-normalized cardiac cycle waveforms of a fixed duration, and first cardiac cycle waveform quality scores 932 according to an embodiment of the invention;

FIG. 18 illustrates a method according to an embodiment of the invention;

FIG. 19 illustrates a method according to an embodiment of the invention;

FIG. 20 illustrates a stage according to an embodiment of the invention; and

FIG. 21 illustrates a stage for calculating a quality of the first detection signals in response to the electrocardiography signals according to an embodiment of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

**DETAILED DESCRIPTION OF THE DRAWINGS**

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings.

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

Measuring oxygen saturation to provide reliable results may be done by illuminating the sternal angle of a user. The sternal angle may be found by the user (or third parties) in a manner such that the user can easily and accurately position the sensor to face sternal angle. This greatly increases the repeatability of obtaining accurate oxygen saturation results. Furthermore - placing the device in this position reduces the breath induced movements that the device experiences and further increases the accuracy of this measurement. In addition-placing the device at that position is relatively easy as the sternum is relatively flat.

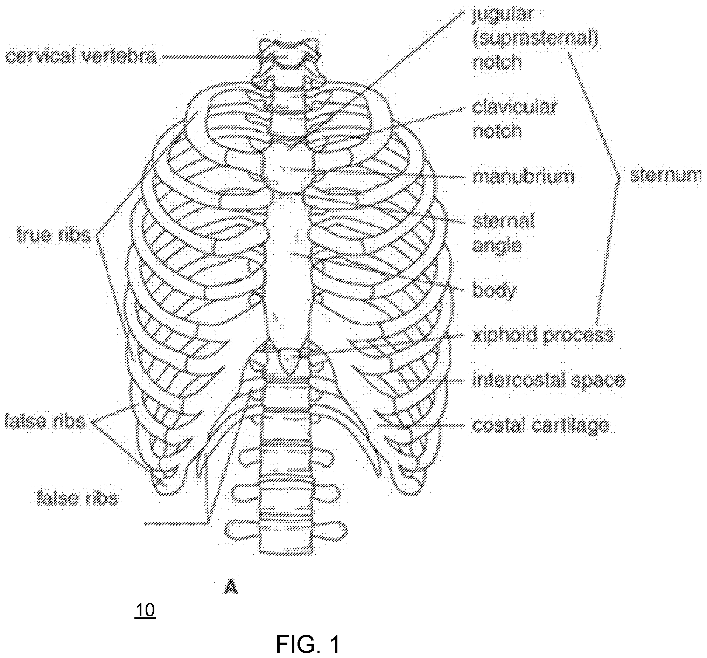

Figure 1 illustrates the sternum and the ribs of a person 10. The sternum angle is located between the manubrium bone and the body of the sternum.

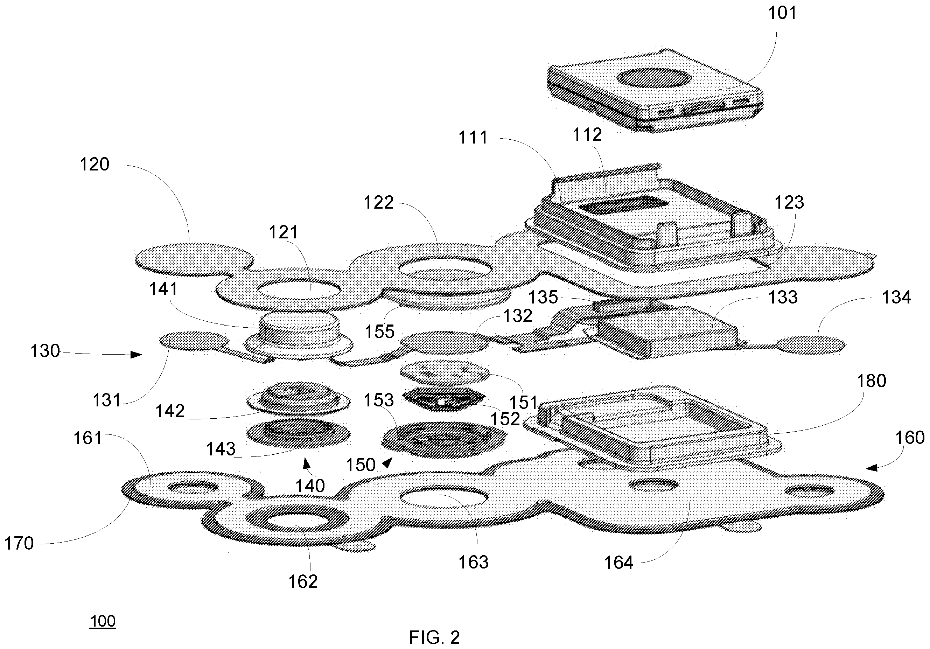

Figure 2 is an exploded view of a vital signs monitoring device 100. The device 100 can be used to measure a plurality of vital signs including, for example, temperature, oxygen saturation, movement, or other. While the device is shown with specific measurement sensors, not all of these need to be used and other sensors could alternatively be used depending on the vital signs it is desirable to measure in given implementation.

In the embodiment shown, the vital signs monitoring device 100 includes:

> 1. Processor and transceiver (collectively denoted 101).
> 2. An upper elastic layer 120 that include first, second and third openings 121, 122 and 123.
> 3. Intermediate layer 130 that includes conductors 131, 132 and 134 and socket 135 for conveying power from battery 133.
> 4. Temperature sensor 140 that includes temperature sensor cover 141, temperature sensor electrical board 142 and temperature sensor case 143.
> 5. Oxygen saturation sensor 150 that includes oxygen saturation sensor electrical board 151, 151, oxygen saturation sensor shield 152 and oxygen saturation sensor case 153.
> 6. A lower elastic layer 160 that include first, second and third openings 161, 162 and 163 and an additional portion 164 to be contacted by lower case
> 7. The lower elastic layer 160 has an underside provided with a self- adhesive. Removable cover 170 shields the self-adhesive and is removed before attaching the device 100 to a user.
> 8. Upper case 111 having socket 112.
> 9. Lower case 180

The temperature sensor cover 141 is shaped and positioned to pass through the first opening 121 of the upper elastic layer 120. Cover 155 is arranged to seal the second opening 122 of the upper elastic layer 120.

Cover 155 is positioned between the upper elastic layer 120 and conductor 132 of the intermediate layer 130. Conductor 132 is positioned above the oxygen saturation sensor electrical board 151.

The temperature sensor case 143 is positioned directly above the first opening 161 of the lower elastic layer 160.

The oxygen saturation sensor 150 is positioned directly above the second opening 162 of the lower elastic layer 160. It may contact the sternum angle during measurements but may be positioned slightly (few millimeters) above the sternum angle without contacting the sternum angle.

Battery 133 is placed within lower case 180 and its upper facet supports a lower facet of upper case 111 that is connected to the processor and transceiver 101.

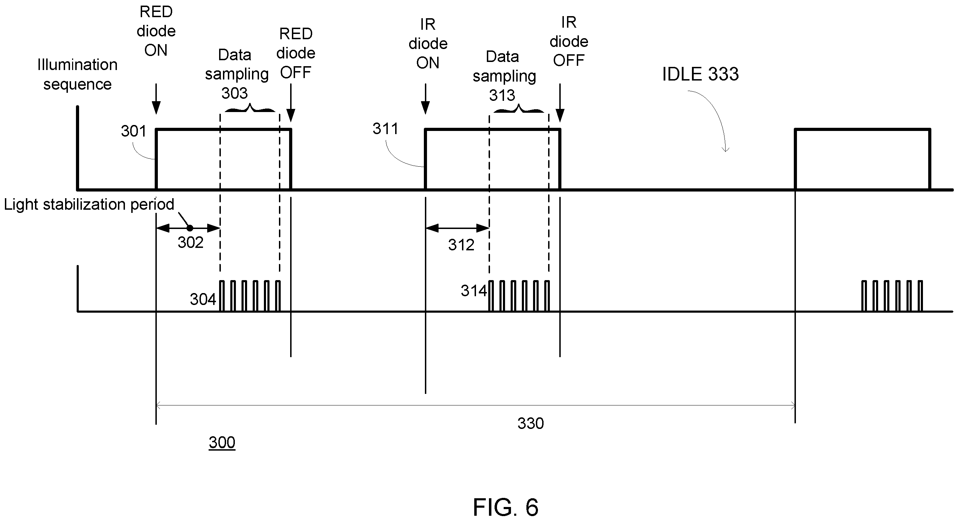

Device 100 is illustrated as including a temperature sensor 140 and oxygen saturation sensor 150. It is noted that other sensor (or sensors) can be provided instead (or in addition) to the temperature sensor 140. Alternatively, the only sensor included in device 100 may be the oxygen saturation sensor 150. For example, as illustrated in figure 6, the device 100 may include a movement sensor 144, a temperature sensor 140, and an oxygen saturation sensor 150.

The device 100 may be very compact and light weight. Its transceiver (denoted 101(2) in figure 6) may be arranged to perform short range and/or long range transmissions.

Device 100 may perform any type of health monitoring. For example, device 100 may measure one or more physiological signals. More specifically, the device 100 may measure one or more vital signs. Device 100 may be equipped with more sensors (and/or different sensors) than illustrated in figure 2.

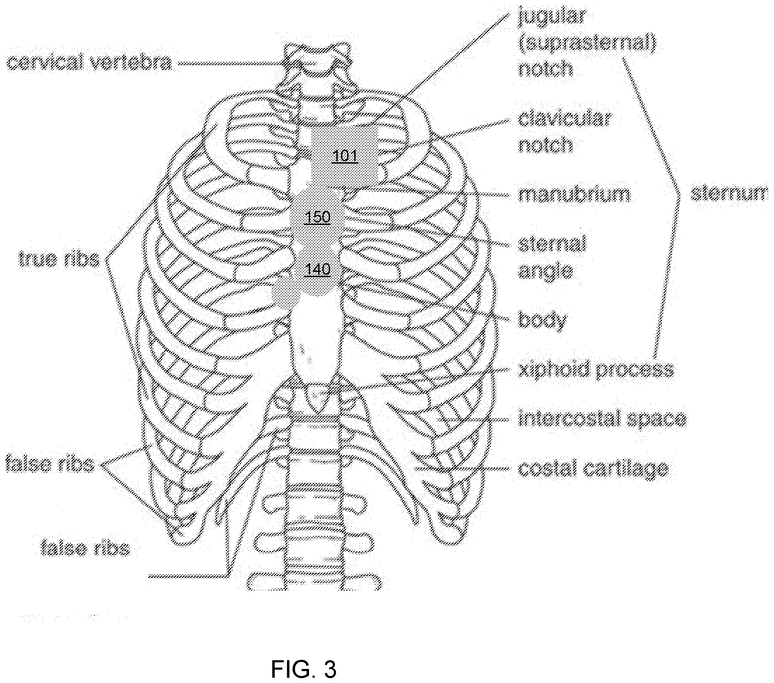

Figure 3 illustrates device 100 as positioned on a user wherein the oxygen saturation sensor 150 is positioned above the sternum angle, the temperature sensor 140 is positioned below the sternum angle and the processor, and the transceiver 101 is positioned above the sternum angle.

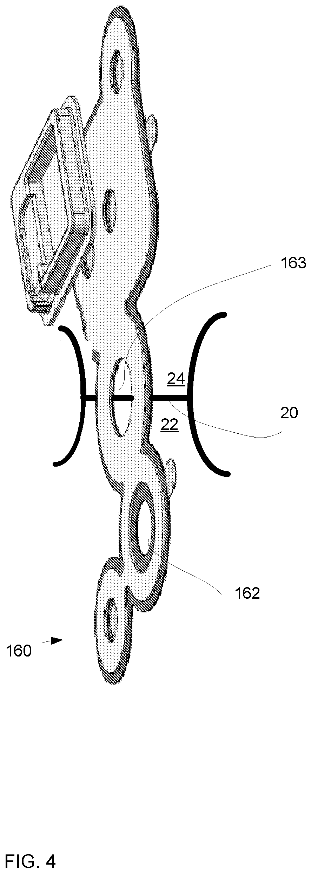

Figure 4 illustrates the lower elastic layer 160 of device 100, sternum angle 20, manubrium bone 24 and sternum body 22. The lower elastic layer 160 of device 100 is positioned above the sternum angle 20, the manubrium bone 24 and the sternum body 22. The third opening 163 (above which the oxygen saturation sensor 150 is positioned) is positioned above the sternum angle 22. The second opening 162 (above which the temperature sensor 140 is positioned) is positioned above the body 24 of the sternum, and the lower case 180 faces the manubrium bone.

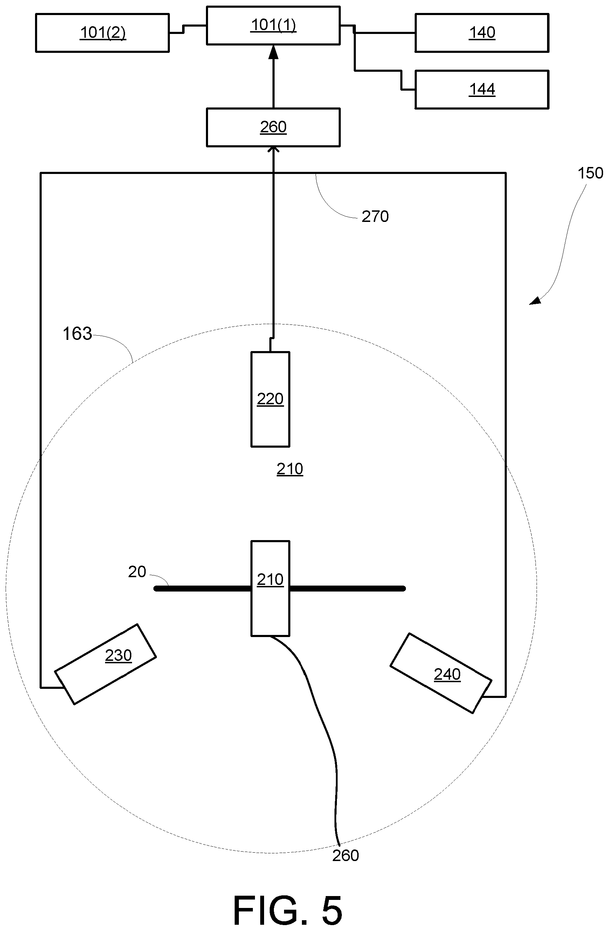

Figure 5 is a schematic diagram of various components of the device 100 of Figure 2.

Figure 5 illustrates the oxygen saturation sensor 150 as including three radiation sensing elements 220, 230 and 240, an illumination module 210 (illustrated as positioned above the sternum angle 20 and within third opening 163 of the lower elastic layer 160 (illustrated in Figure 2)), an intermediate module 260 (that may include an analog amplifier, an analog to digital converter or a combination of both), a processor 101(1) of processor/transducer 101 (illustrated in Figure 2), a transducer 101(2), a temperature sensor 140, and a movement sensor 144.

The illumination module 210 may be arranged to illuminate the sternum angle with infrared pulses and visible light pulses. The radiation sensing elements 220, 230 and 240 may sense radiation reflected and/or scattered from the sternum angle in the infrared and visible light ranges and send detection signals towards intermediate module 260. The illumination module 210 may include one or more IR light sources such as one or more IR diodes, and may include one visible light sources such as one or more visible light diodes

Pulses of energy are provided (from an energy source that is not shown) to the illumination module 210 via a conductor 270 Radiation sensing elements 220, 230 and 240 are coupled in parallel to each other via conductor 270. In alternative embodiments, the radiation sensing elements 22, 230, and 240 may be coupled to one another in a serial manner or in any other suitable manner.

Processor 101(1) may receive detection signals from temperature sensor 140 and movement sensor 144. It may be arranged to disregard detection signals obtained when the user moves in a manner that may reduce the reliability of the detection signals below a predefined threshold.

Oxygen saturation measurement using the device 100 is done with the oxygen saturation sensor 150. In order to measure oxygen saturation, however, illumination of the sternal angle of the patient must be done. Figure 6 is a timing diagram 300 of such illumination and measurement according to an embodiment of the invention. It illustrates a cyclic illumination pattern having a period of 330 during which the sternal angle is illuminated and measurements are taken. Each cycle includes an activation window 301 of a red diode (delimited between RED diode ON and RED diode OFF) and an activation window 311 of an infrared diode (delimited between IR diode ON and IR diode OFF) that are followed by an idle period 333. Each activation window includes a stabilization period (302 and 312 respectively) in which the emitted light (red or infrared) is stabilized. The stabilization period (302 or 312) that is followed by a measurement period (303 and 313) in which the light pulses (304 and 314 respectively) can be used for oxygen saturation measurements. The activation windows may be of the same length (for example 0.5 millisecond) or of different lengths. The cyclic illumination pattern may have a cycle 330 that is longer and even much longer than the duration of the activation windows (for example - 13 millisecond). It should be noted that measurement periods 303 and 313 may be a part of activation windows 301 and 313 respectively.

Detection signals generated during idle period 333 may be indicative of unwanted ambient light and may be discarded.

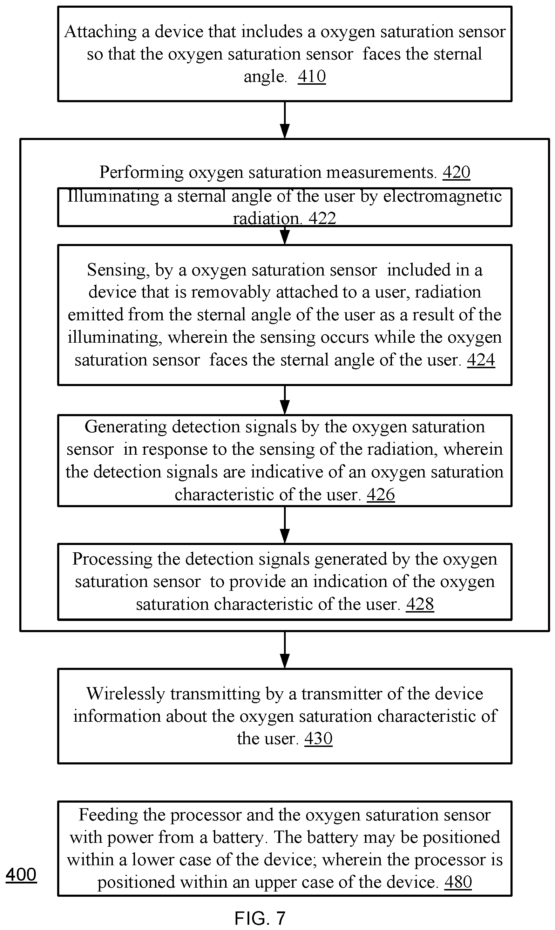

Figure 7 illustrates a method 400 for providing an indication of the oxygen saturation characteristic of the user according to an embodiment of the invention. Method 400 may be performed at least in part using the vital signs monitoring device 100 of Figure 2. When description is made of specific device features in executing the method, reference should be made to Figure 2. It is to be appreciated that in alternative embodiments method 400 may be executed using a device other than device 100 so long as the device has the necessary components for executing the steps of the method 400.

Method 400 may start with stage 410 of attaching a device that includes an Oxygen saturation sensor so that the oxygen saturation sensor faces the sternal angle. This may, for example, include positioning device 100 (or any other device that has an oxygen saturation sensor for sensing oxygen saturation characteristics) on a user. The device can be attached using a self-adhesive material, using a belt, or using any other suitable fixation means. The Oxygen saturation sensor may be Oxygen saturation sensor 150 of figure 1.

Stage 410 may be followed by stage 420 of performing oxygen saturation measurements. Multiple oxygen saturation measurements can be performed over short or long periods of time. Such periods of time may be minutes, hours, days and even more.

An oxygen saturation measurement may include a detection signal acquisition phase and a processing phase. The detection signal acquisition phase is executed by the device attached to the client. The processing stage can be executed in full by the device, can be partially executed by the device, or can be executed by another device or system not attached to the device.

The detection signal acquisition stage may include the process described in association with the timing diagram 300 of Figure 6. The detection signal acquisition stage may include:

> 1. Illuminating (stage 422) a sternal angle of the user. In some embodiments such illumination may be by electromagnetic radiation but other types of illumination may be used. The illumination may be done, for example, by the illumination module 210 of figure 5.
> 2. Sensing (stage 424) by an oxygen saturation sensor included in a device that is removably attached to a user, radiation emitted from the sternal angle of the user. The radiation detected can result from the illuminating of the sternal angle. The sensing occurs while the oxygen saturation sensor faces the sternal angle of the user. The Oxygen saturation sensor may be Oxygen saturation sensor 150 of figure 1.
> 3. Generating detection signals (stage 426) by the oxygen saturation sensor in response to the sensing of the radiation, wherein the detection signals are indicative of an oxygen saturation characteristic of the user.

Stage 422 may include illuminating the sternal angle of the user by a diode that emits visible light pulses and infrared pulses in an interleaved manner. The diode may belong to illumination module 210 of figure 5. Stage 422 may be executed by an illumination module of the device. The illumination module may be the illumination module 210 of figure 5.

Stage 424 may include sensing the radiation by one or more sensing elements such as photodiodes. If there are multiple sensing elements the sensing elements may be coupled to each other in parallel, in serial, or a combination thereof. The sensing can be executed by three radiation sensing elements 220, 230 and 240 of figure 5.

Stage 424 may include sensing the radiation using a plurality of photodiodes that are arranged in a radially symmetrical manner.

The processing phase includes processing (stage 428) the detection signals generated by the oxygen saturation sensor to provide an indication of the oxygen saturation characteristic of the user.

If the processing is performed by a processor of the device then stage 428 Is preceded (or includes) sending the detection signals to the processor of the device. If the processing is executed by a processor that does not belong to the device then the method includes transmitting the detection signals towards that processor.

Stage 420 may be followed by stage 430 of wirelessly transmitting by a transmitter of the device information about the oxygen saturation characteristic of the user.

Method 400 may also include stage 480 of feeding the processor 101 and the oxygen saturation sensor 150 with power from a battery 133, reference being made to elements of Figure 2 to illustrate an embodiment of a device for use with method 400. As shown in that figure, the battery 130 may be positioned within a lower case 180 of the device 100. The processor 101 may be positioned within an upper case of the device 111.

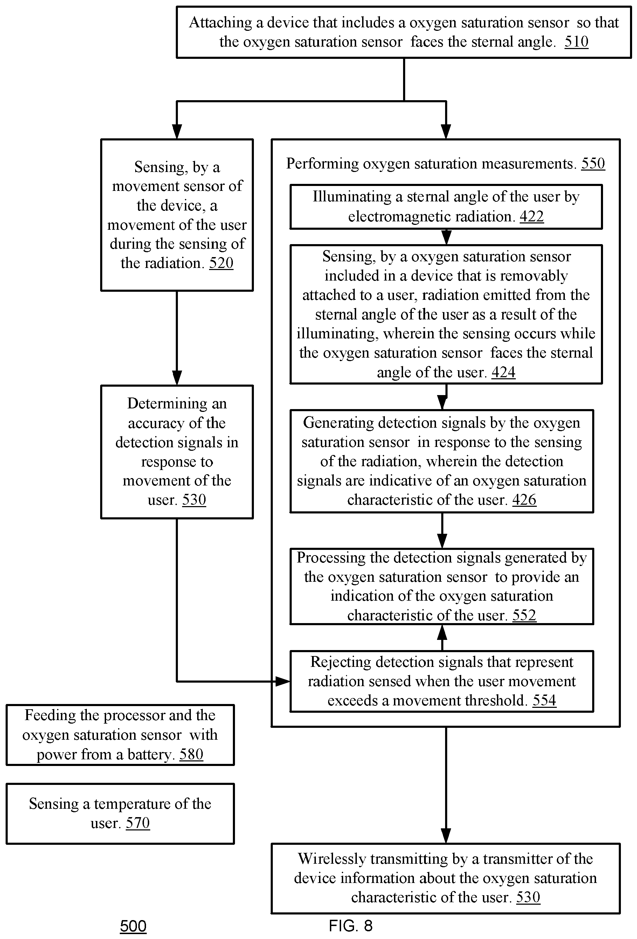

Figure 8 illustrates method 500 according to an embodiment of the invention. Method 500 may be performed at least in part using the vital signs monitoring device 100 of Figure 2. When description is made of specific device features in executing the method, reference should be made to Figure 2. It is to be appreciated that in alternative embodiments method 500 may be executed using a device other than device 100 so long as the device has the necessary components for executing the steps of the method 500.

Method 500 starts by stage 510 of attaching a device that includes an oxygen saturation sensor so that the oxygen saturation sensor faces the sternal angle. For example, stage 510 may include positioning device 100 of Figure 2 such that the oxygen saturation sensor 150 faces the sternal angle.

Stage 510 may be followed by stages 520 and 550.

Stage 520 may include sensing, by a movement sensor of the device, a movement of the user during the sensing of the radiation. The movement sensor may be, for example, movement sensor 144 described with respect to Figure 5.

Stage 520 may be followed by stage 530 of determining an accuracy of the detection signals in response to movement of the user. Higher accuracy levels are obtained when smaller movements are detected.

Stage 550 may include performing oxygen saturation measurements. Multiple oxygen saturation measurements can be performed over short or long periods of time- minutes, hours, days and even more. Stage 550 may include stages 422, 424 and 426, stages 422, 424, and 426 being substantially as described with respect to Figure 7. Stage 550 may also include stage 552 of processing the detection signals by the oxygen saturation sensor to provide an indication of the oxygen saturation characteristic of the user and stage 554 of rejecting detection signals that represent radiation sensed when the user movement exceeds a movement threshold.

If the processing is performed by a processor of the device then stage 552 is preceded (or includes) sending the detection signals to the processor of the device. If the processing is executed by a processor that does not belong to the device then the method includes transmitting the detection signals towards that processor.

Stage 550 may be followed by stage 560 of wirelessly transmitting by a transmitter of the device information about the oxygen saturation characteristic of the user.

Method 500 may also include stage 580 of feeding the processor 101 and the oxygen saturation sensor 150 with power from a battery 133, reference being made to elements of Figure 2 to illustrate an embodiment of a device for use with method 500. As shown in that figure, the battery 130 may be positioned within a lower case 180 of the device 100. The processor 101 may be positioned within an upper case of the device 111.

Figure 8 also illustrates method 500 as sensing (570) a temperature of the user by a temperature sensor 140 of the device 100 (see Figure 2). It is noted that this stage can include performing any further sensing operation by any other type of sensor.

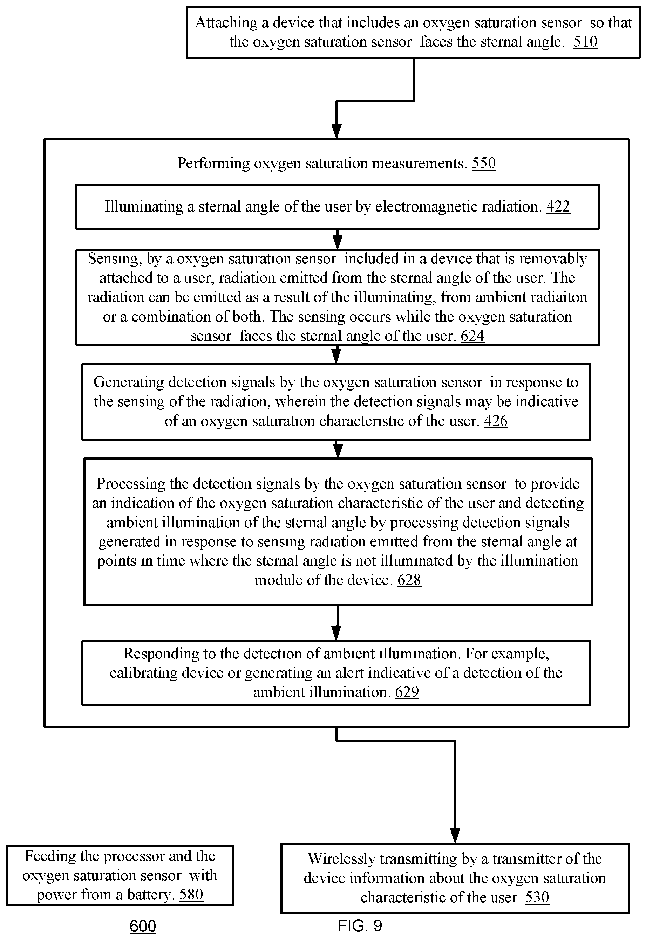

Figure 9 illustrates method 600 according to an embodiment of the invention. Method 600 may be performed at least in part using the vital signs monitoring device 100 of Figure 2. When description is made of specific device features in executing the method, reference should be made to Figure 2. It is to be appreciated that in alternative embodiments method 600 may be executed using a device other than device 100 so long as the device has the necessary components for executing the steps of the method 600.

Method 600 may start with stage 610 of attaching a device that includes an oxygen saturation sensor so that the oxygen saturation sensor faces the sternal angle. For example, stage 610 may include positioning device 100 of Figure 2 such that the oxygen saturation sensor 150 faces the sternal angle.

Stage 610 may be followed by stage 620 of performing oxygen saturation measurements. The oxygen saturation measurements may involve Oxygen saturation sensor 150 of figure 5.

An oxygen saturation measurement may include a detection signal acquisition phase and a processing phase. The detection signal acquisition phase is executed by the device attached to the client. The processing stage can be executed in full by the device, can be partially executed by the device or can be executed by another device or system not attached to the device.

The detection signal acquisition stage includes:

> 1. Illuminating (stage 422) a sternal angle of the user by electromagnetic radiation. This can be done by illumination module 210 of figure 5.
> 2. Sensing (stage 624), by an oxygen saturation sensor included in a device that is removably attached to a user, radiation emitted from the sternal angle of the user. The radiation detected can result of the illuminating of the sternal angle, from ambient illumination of from a combination thereof. The sensing occurs while the oxygen saturation sensor faces the sternal angle of the user. This can be done by the three radiation sensing elements 220, 230 and 240 of figure 5.
> 3. Generating detection signals (stage 426) by the oxygen saturation sensor in response to the sensing of the radiation, wherein the detection signals are indicative of an oxygen saturation characteristic of the user. This can be done by the three radiation sensing elements 220, 230 and 240 of figure

Stage 624 may include sensing the radiation by one or more sensing elements such as photodiodes. If there are multiple sensing elements the sensing elements may be coupled to each other in parallel, in serial or a combination thereof. It should be noted that while step 424 sensed radiation detected only as a result of the illumination of the sternal angle by the illumination unit - step 624 may sense radiation detected as a result of the illuminating of the sternal angle, from ambient illumination of from a combination thereof.

The processing phase includes processing (stage 628) the detection signals by the oxygen saturation sensor to provide an indication of the oxygen saturation characteristic of the user.

Stage 628 may include detecting ambient illumination of the sternal angle by processing detection signals generated (during stage 426) in response to sensing radiation emitted from the sternal angle at points in time where the sternal angle is not illuminated by the illumination module of the device. See, for example, generation of detection signals that sense ambient radiation sensed during idle period 333 of Figure 5.

Stage 628 may be followed by stage 629 of responding to the detection of ambient illumination.

For example, such responding may include calibrating the device or generating an alert indicative of a detection of the ambient illumination. The calibrating may include estimating the ambient light and compensating the oxygen saturation measurements in response to the ambient light. For example- reducing from detected radiation (detected when illuminating the sternum angle by IR or light pulse) the estimated value of the ambient light (IR component or light component respectively).

The alert may signal the user that he should re-attach the device in order to reduce or eliminate ambient radiation from reaching the sternum angle.

If the processing is performed by a processor of the device then stage 628 is preceded (or includes) sending the detection signals to the processor of the device. If the processing is executed by a processor that does not belong to the device then the method includes transmitting the detection signals towards that processor.

Stage 620 may be followed by stage 630 of wirelessly transmitting by a transmitter of the device information about the oxygen saturation characteristic of the user.

Method 600 may also include stage 680 of feeding the processor 101 and the oxygen saturation sensor 150 with power from a battery 133, reference being made to elements of Figure 2 to illustrate an embodiment of a device for use with method 600. As shown in that figure, the battery 130 may be positioned within a lower case 180 of the device 100. The processor 101 may be positioned within an upper case of the device 111.

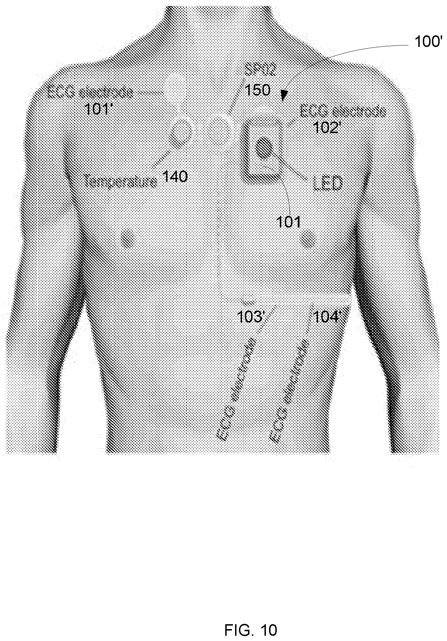

Figure 10 illustrates a vital signs monitoring device 100' that is removably attached to a person.

The vital signs monitoring device 100' has a temperature sensor 140, an oxygen saturation sensor 150, a processor and transceiver 101. In some embodiments, the device 100' may be substantially the same as the device 100 illustrated in previous figures. It is to be appreciated, however, that other configurations could be used.

Device 100' may include one or multiple electrocardiography (ECG) electrodes such as electrodes 101', 102', 103' and 104'.

It is desirable to aim the oxygen saturation sensor 150 of the device 100' to illuminate the sternal angle of the person. This can be done by performing a positioning process.

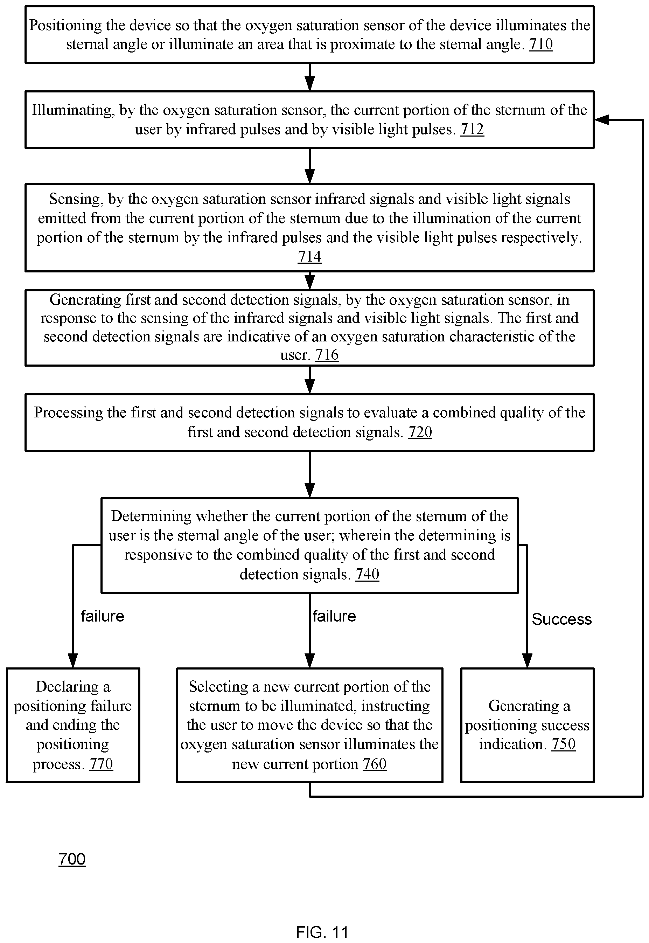

Figure 11 illustrates a method 700 of positioning a vital signs monitoring device, such as the device 100 of Figure 2 or the device 100' of Figure 10, according to an embodiment of the invention. When description is made of specific device features in executing the method, reference should be made to Figures 2 and 10. It is to be appreciated that in alternative embodiments method 700 may be executed using a device other than device 100 or 100' so long as the device has the necessary components for executing the steps of the method 600.

Method 700 may start with stage 710 of positioning the device 100, 100' so that the oxygen saturation sensor 150 of the device illuminates the sternal angle or illuminates an area that is proximate (for example by less than 10 centimeters) to the sternal angle. It may be assumed that the device 100, 100' is positioned so that the oxygen saturation sensor 150 illuminates a current portion of the sternum of the user.

During a first execution of stage 710 the current portion is a first portion.

Stage 710 is followed by stage 712 of illuminating, by the oxygen saturation sensor 150, the current portion of the sternum of the user by infrared pulses and by visible light pulses. Pulses of different wavelength (infrared and visible light) may be transmitted towards the current portion of the sternum in a non-overlapping manner (at different points of time).

Stage 712 may be followed by stage 714 of sensing, by the oxygen saturation sensor 150, infrared signals and visible light signals emitted from the current portion of the sternum due to the illumination of the current portion of the sternum by the infrared pulses and the visible light pulses respectively.

Stage 714 may be followed by stage 716 of generating first and second detection signals, by the oxygen saturation sensor, in response to the sensing of the infrared signals and visible light signals. The first and second detection signals are indicative of an oxygen saturation characteristic of the user. The oxygen saturation sensor may be the oxygen saturation sensor 150 of figure 5.

The first detection signals are responsive to the infrared signals and the second detection signals are responsive to the visible light signals.

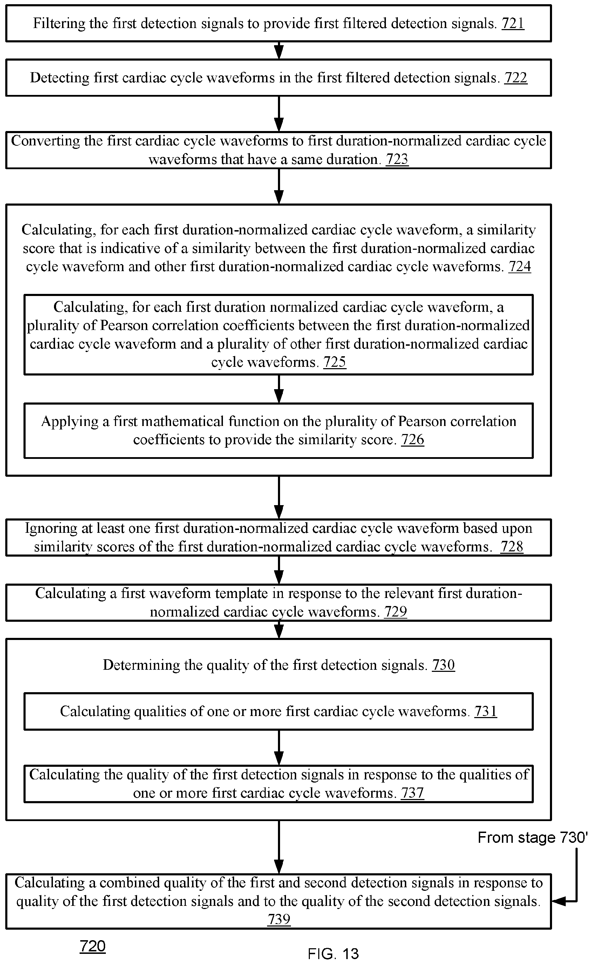

Stage 716 may be followed by stage 720 of processing the first and second detection signals to evaluate a quality of the first and second detection signals. Further description of such processing is given below in the description of Figures 13-15.

Stage 720 may be followed by stage 740 of determining whether the current portion of the sternum of the user is the sternal angle of the user; wherein the determining is responsive to the quality of the first and second detection signals.

Stage 740 may include determining that the current portion of the sternum of the user is the sternal angle of the user if the quality of the first and second detection signals exceeds a predetermined quality threshold.

Stage 720 and/or step 740 may be executed by the oxygen saturation sensor, by a computerized device that includes the oxygen saturation sensor, or by a computerized device that does not include the oxygen saturation sensor or may be executed in part by the oxygen saturation sensor and in part by the computerized device that does not include the oxygen saturation sensor.

If it is determined that the current portion of the sternum of the user is the sternal angle of the user, then stage 740 may be followed by stage 750 of generating a positioning success indication.

The positioning success indication may be sent to the user, to a user device, or to a third party. The aim of the positioning success indication is to notify the user or a third party that the device is positioned such that the oxygen saturation sensor illuminates the sternal angle of the user and that the device may now be fixed in place. Such fixing may include peeling a protective element and detachably connecting the device to the user.

If it is determined that the current portion of the sternum of the user is not the sternal angle of the user, then stage 740 may be followed by stage 760 of selecting a new current portion of the sternum to be illuminated, instructing the user to move the device so that the oxygen saturation sensor illuminates the new current portion and repeating stages 712, 714, 716, 720 and 740 for the new current portion.

It is also noted that if it is determined that the current portion of the sternum of the user is not the sternal angle of the user, then stage 740 may be followed by stage 770 of declaring a positioning failure and ending the positioning process.

According to another embodiment of the invention, stages 712, 714, 716, 720, 740 and 760 are repeated multiple times to find one or more current portions of the sternum that are valid candidates of a sternal angle. The best current portion of the one or more valid candidates may then be selected. For example, selecting the valid candidate with the highest quality. Each valid candidate may have a quality that exceeds a valid candidate quality threshold. The valid candidate quality threshold may not exceed the predetermined quality threshold.

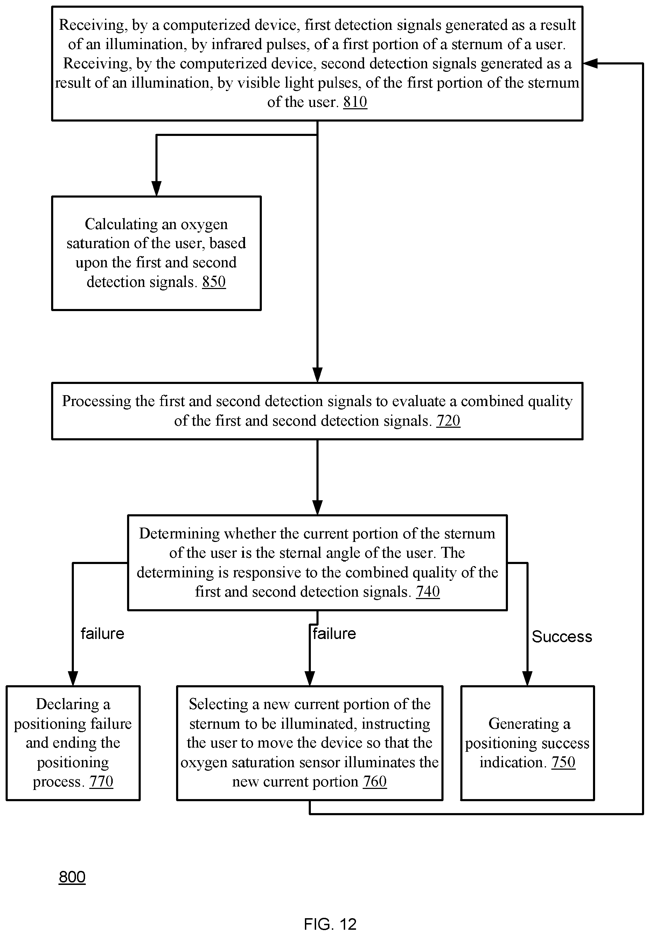

Figure 12 illustrates a method 800 of evaluating the position of a vital signs monitoring device, such as device 100 of Figure 2 or device 100' of Figure 10, according to an embodiment of the invention. When description is made of specific device features in executing the method, reference should be made to Figures 2 and 10. It is to be appreciated that in alternative embodiments method 700 may be executed using a device other than device 100 or 100' so long as the device has the necessary components for executing the steps of the method 600.

Method 800 is executed by a computerized device. As is described more fully below, method 800 may include stages 720, 740, 750, 760, and 770. These stages are substantially as described with respect to the same stages in Figure 11.

Method 800 starts with stage 810 of (a) receiving, by a computerized device, first detection signals generated as a result of an illumination, by infrared pulses, of a first portion of a sternum of a user; and (b) receiving, by the computerized device, second detection signals generated as a result of an illumination, by visible light pulses, of the first portion of the sternum of the user;

Stage 810 is followed by stage 720 of processing the first and second detection signals to evaluate a quality of the first and second detection signals.

Stage 720 may be followed by stage 740 of determining whether the current portion of the sternum of the user is the sternal angle of the user. The determining may be responsive to the quality of the first and second detection signals. Stage 740 may be followed by stage 750, 760 or 770.

Stage 810 may be followed by stage 850 of calculating an oxygen saturation of the user, based upon the first and second detection signals. Stage 850 may be executed in parallel to any one of stages 720, 740, 750, 770 and 760, may be executed before or after any one of stages 720, 740, 750, 770 and 760.

Differences between amplitudes of infrared signals and visible light signals emitted from the user are indicative of the oxygen saturation of the user. Specifically, the ratio between the amplitudes of infrared signals and the visible light signals detected by the oxygen saturation sensor is indicative of the oxidation level of the blood of the user. Step 850 may include calculating these differences.

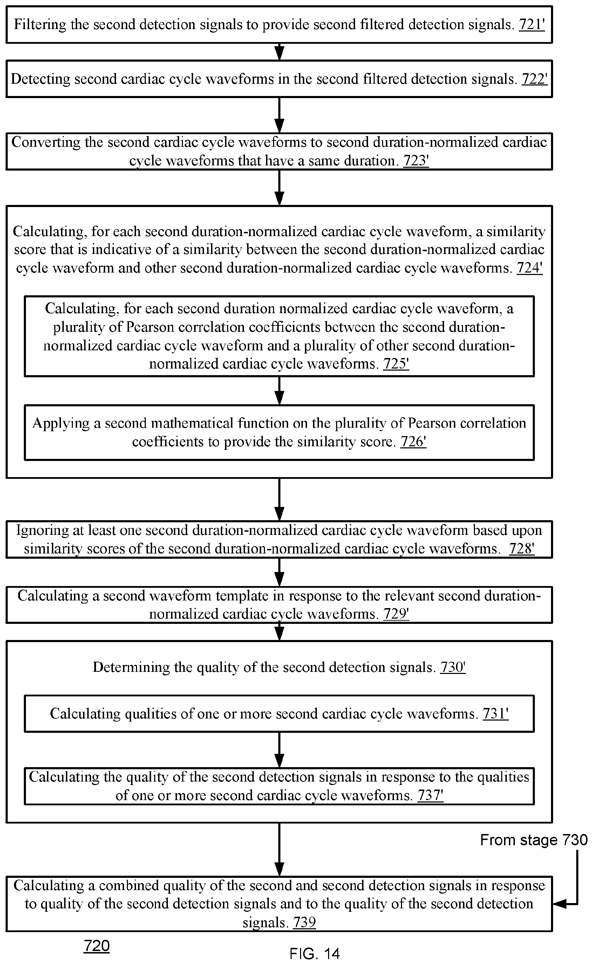

Figures 13-15 illustrate stage 720 of processing the first and second detection signals to evaluate a quality of the first and second detection signals according to an embodiment of the invention. Figure 13 illustrates determining the quality of the first detection signal and Figure 14 illustrates determining the quality of the second detection signal. In the final stage of Figures 13 and 14, stage 739, a combined quality of the first and second detection signals is calculated.

Stage 720 may include at least one of the stages described below. While in some embodiments, stage 720 may include all of the described stages, in other embodiments one more of the described stages may not be included in stage 720.

Turning first to Figure 13, addressing the first filtered detection signals, stage 720 may start with stage 721 including filtering the first detection signals (from stage 716 of Figure 11) to provide first filtered detection signals. The filtering may include high-pass filtering and low-pass filtering or applying bandpass filtering. The low-pass filtering may be bilateral filtering, any other edge preserving filtering or any other filtering.

Stage 721 may be followed by stage 722 of detecting first cardiac cycle waveforms in the first filtered detection signals.

Stage 722 may be followed by stage 723 of converting the first cardiac cycle waveforms to first duration-normalized cardiac cycle waveforms that have a same duration.

Stage 723 may be followed by stage 724 of calculating, for each first duration-normalized cardiac cycle waveform, a similarity score that is indicative of a similarity between the first duration- normalized cardiac cycle waveform and other first duration-normalized cardiac cycle waveforms.

Stage 724 may include stage 725 of calculating, for each first duration normalized cardiac cycle waveform, a plurality of Pearson correlation coefficients between the first duration-normalized cardiac cycle waveform and a plurality of other first duration-normalized cardiac cycle waveforms. The plurality of other first duration-normalized cardiac cycle waveforms may include all of the first duration-normalized cardiac cycle waveforms that differ from the first duration normalized cardiac cycle waveform or only some of these other first duration-normalized cardiac cycle waveforms.

For example, a Pearson correlation coefficient (Rij) between an i'th first duration-normalized cardiac cycle waveform (wi) and a j'th first duration-normalized cardiac cycle waveform (wj) may be expressed by the following equation:

$$Ri,j = \text{covariance } (wi, wj)/ \text{std}(wi)*\text{std}(wj).$$

Wherein "std" stands for a standard deviation

Stage 725 may be followed by stage 726 of applying a first mathematical function on the plurality of Pearson correlation coefficients to provide the similarity score. It is to be appreciated that stage 726 may be a part of stage 724. The applying may include, for example, summing the plurality of Pearson correlation coefficients to provide the similarity score.

Stage 724 may be followed by stage 728 of ignoring at least one first duration-normalized cardiac cycle waveform based upon similarity scores of the first duration-normalized cardiac cycle waveforms. Stage 728 provides relevant first duration- normalized cardiac cycle waveforms (those first duration-normalized cardiac cycle waveform that were not ignored).

Stage 728 may include, for example, ignoring one or more first duration-normalized cardiac cycle waveform that have a similarity score that is below a similarity score threshold, or ignoring a preset number of first duration-normalized cardiac cycle waveforms that have the lowest similarity scores.

Stage 728 may be followed by stage 729 of calculating a first waveform template in response to the relevant first duration-normalized cardiac cycle waveforms. This stage may include applying a second mathematical function on the relevant first duration-normalized cardiac cycle waveforms. The second mathematical function may be any mathematical function. If may be, for example, a weighted averaging function, an averaging function, or other mathematical function.

Stage 729 may be followed by stage 730 of determining the quality of the first detection signals.

Stage 730 may include stage 731 of calculating qualities of one or more first cardiac cycle waveforms. These one or more first cardiac cycle waveforms may include all the first cardiac cycle waveforms detected during stage 722 or only some of the first cardiac cycle waveforms detected during stage 722. For example, the one or more first cardiac cycle waveforms may correspond to the relevant first duration- normalized cardiac cycle waveforms.

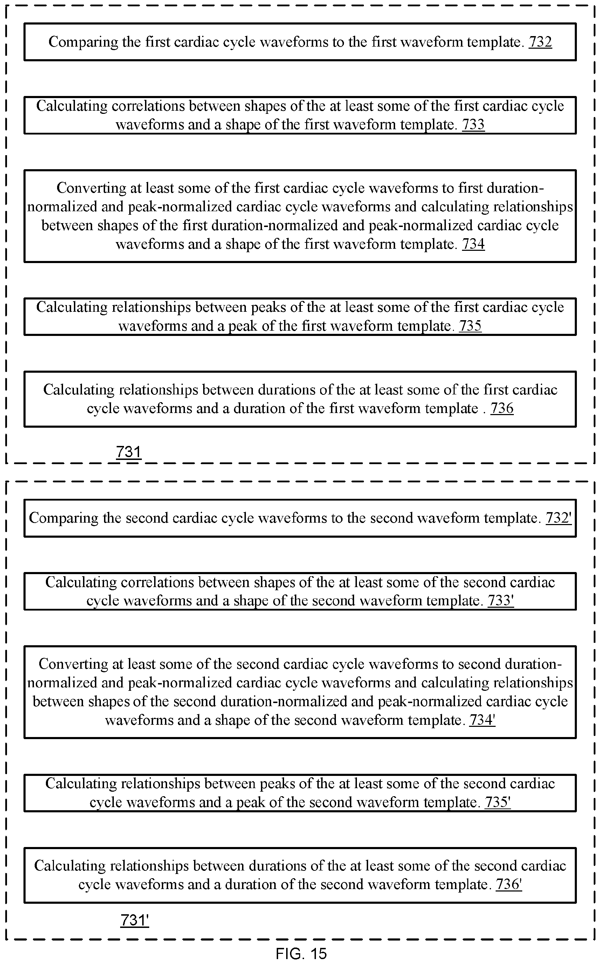

Reference is now made to Figure 15 for more detail regarding stage 731. Stage 731 may include at least one of stages 732, 733, 734, 735 and 736. For example, stage 731 may include stages 734, 735 and 736.

Stage 732 may include comparing the first cardiac cycle waveforms to the first waveform template.

Stage 733 may include calculating correlations between shapes of the at least some of the first cardiac cycle waveforms and a shape of the first waveform template.

Stage 734 may include converting at least some of the first cardiac cycle waveforms to first duration-normalized and peak-normalized cardiac cycle waveforms and calculating relationships between shapes of the first duration-normalized and peak- normalized cardiac cycle waveforms and a shape of the first waveform template. The first duration-normalized and peak-normalized cardiac cycle waveforms are a same duration and a same peak value as the first waveform template.

Stage 735 may include calculating relationships between peaks of the at least some of the first cardiac cycle waveforms and a peak of the first waveform template.

Stage 736 may include calculating relationships between durations of the at least some of the first cardiac cycle waveforms and a duration of the first waveform template quality of the first detection signals.

Returning now to Figure 13, stage 730 may include stage 737 of calculating the quality of the first detection signals in response to the qualities (calculated during stage 731) of one or more first cardiac cycle waveforms.

Turning now to Figure 14, addressing the second filtered detection signals, stage 721' may include filtering the second detection signals to provide second filtered detection signals. The filtering may include high-pass filtering and low-pass filtering or applying bandpass filtering. The low-pass filtering may be bilateral filtering, any other edge preserving filtering or any other filtering.

Stage 721' may be followed by stage 722' of detecting second cardiac cycle waveforms in the second filtered detection signals.

Stage 722' may be followed by stage 723' of converting the second cardiac cycle waveforms to second duration-normalized cardiac cycle waveforms that have a same duration.

Stage 723' may be followed by stage 724' of calculating, for each second duration-normalized cardiac cycle waveform, a similarity score that is indicative of a similarity between the second duration-normalized cardiac cycle waveform and other second duration-normalized cardiac cycle waveforms.

Stage 724' may include stage 725' of calculating, for each second duration normalized cardiac cycle waveform, a plurality of Pearson correlation coefficients between the second duration-normalized cardiac cycle waveform and a plurality of other second duration-normalized cardiac cycle waveforms. The plurality of other second duration-normalized cardiac cycle waveforms may include all of the second duration- normalized cardiac cycle waveforms that differ from the second duration normalized cardiac cycle waveform or only some of these other second duration-normalized cardiac cycle waveforms.

Stage 725' may be followed by stage 726' of applying a first mathematical function on the plurality of Pearson correlation coefficients to provide the similarity score. It is to be appreciated that stage 726' may be included in stage 724'. The applying may include, for example, summing the plurality of Pearson correlation coefficients to provide the similarity score.

Stage 724' may be followed by stage 728' of ignoring at least one second duration-normalized cardiac cycle waveform based upon similarity scores of the second duration-normalized cardiac cycle waveforms. Stage 728' provides relevant second duration-normalized cardiac cycle waveforms (those second duration-normalized cardiac cycle waveform that were not ignored).

Stage 728' may include, for example, ignoring one or more second duration-normalized cardiac cycle waveform that have a similarity score that is below a similarity score threshold, or ignoring a preset number of second duration-normalized cardiac cycle waveforms that have the lowest similarity scores.

Stage 728' may be followed by stage 729' of calculating a second waveform template in response to the relevant second duration-normalized cardiac cycle waveforms. This stage may include applying a second mathematical function on the relevant second duration-normalized cardiac cycle waveforms. The second mathematical function may be any mathematical function. If may be, for example. A weighted averaging function, an averaging

Stage 729' may be followed by stage 730' of determining the quality of the second detection signals.

Stage 730' may include stage 731' of calculating qualities of one or more second cardiac cycle waveforms. These one or more second cardiac cycle waveforms may include all the second cardiac cycle waveforms detected during stage 722' or only some of the second cardiac cycle waveforms detected during stage 722'. For example, the one or more second cardiac cycle waveforms may correspond to the relevant second duration-normalized cardiac cycle waveforms.

Reference is now made to Figure 15 for more detail regarding stage 731'. Stage 731' may include at least one out of stages 732', 733', 734', 735' and 736'. For example, stage 731' may include stages 734, 735' and 736'.

Stage 732' may include comparing the second cardiac cycle waveforms to the second waveform template.

Stage 733' may include calculating correlations between shapes of the at least some of the second cardiac cycle waveforms and a shape of the second waveform template.

Stage 734' may include converting at least some of the second cardiac cycle waveforms to second duration-normalized and peak-normalized cardiac cycle waveforms and calculating relationships between shapes of the second duration- normalized and peak-normalized cardiac cycle waveforms and a shape of the second waveform template. The second duration-normalized and peak-normalized cardiac cycle waveforms are a same duration and a same peak value as the second waveform template.

Stage 735' may include calculating relationships between peaks of the at least some of the second cardiac cycle waveforms and a peak of the second waveform template.

Stage 736' may include calculating relationships between durations of the at least some of the second cardiac cycle waveforms and a duration of the second waveform template quality of the second detection signals.

Returning now to Figure 13, stage 730' may include stage 737' of calculating the quality of the second detection signals in response to the qualities (calculated during stage 731') of one or more second cardiac cycle waveforms.

As shown in each of Figures 13 and 14, stages 730 and 730' may be followed by stage 739 of calculating a quality of the first and second detection signals in response to quality of the first detection signals and to the quality of the second detection signals. Stage 739 may include summing, weighted summing, averaging or applying any function on the quality of the first detection signals and the quality of the second detection signals.

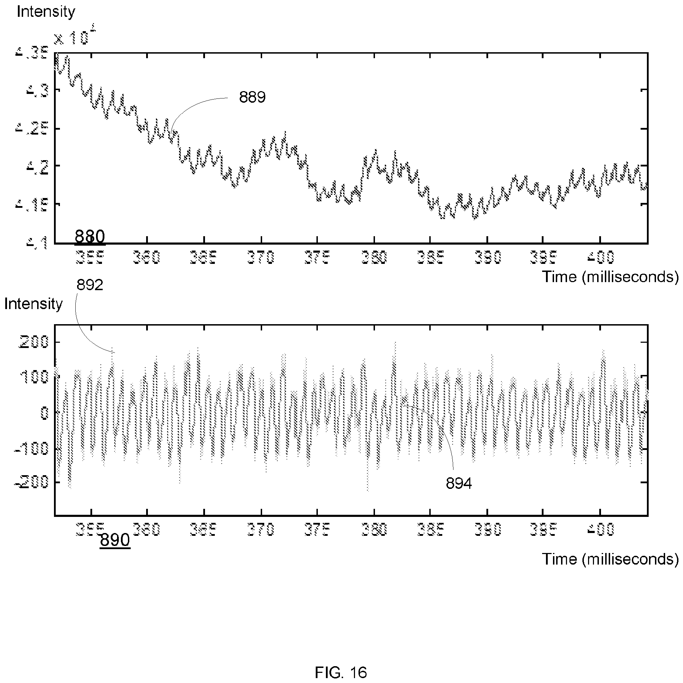

Figure 16 illustrates first detection signals 882 and first filtered detection signals 892 according to an embodiment of the invention.

Graph 880 of figure 16 illustrates first detection signals 882.

Graph 890 of figure 16 illustrates first filtered detection signals 892 and 894. First filtered detection signals 892 were filtered only by a high-pass filter (a Butterworth high-pass filter) while first filtered detection signals 894 were filtered using both a high-pass filter and a low-pass (Bilateral) filter.

The x-axis of graphs 880 and 890 represent time while the y-axis of graphs 880 and 890 represent intensity.

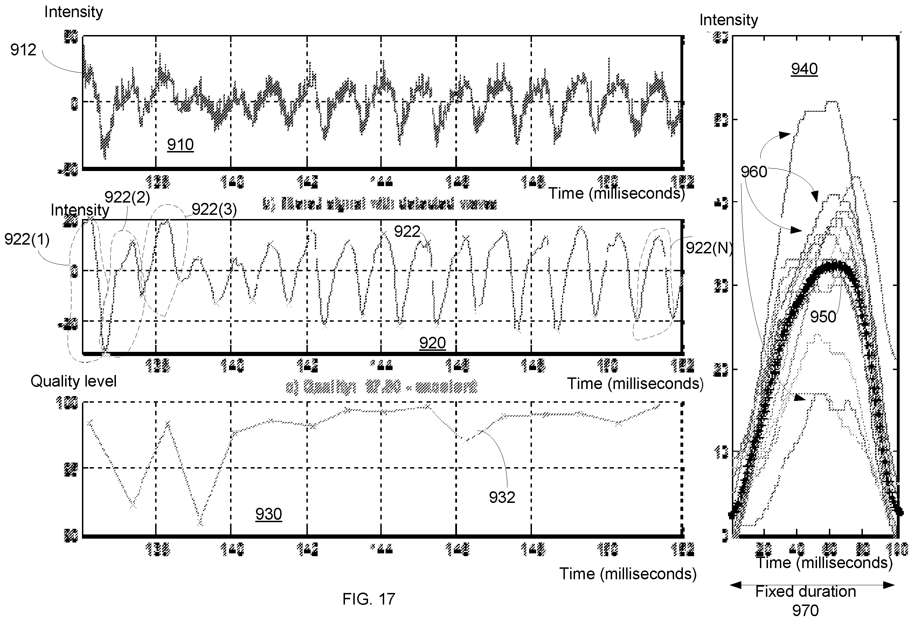

Figure 17 illustrates first detection signals 912, first filtered detection signals 922, first cardiac cycle waveforms 922(1)- 922(N), first waveform template 950 and first duration-normalized cardiac cycle waveforms 960 of a fixed duration 970, and first cardiac cycle waveform quality scores 932 according to an embodiment of the invention.

Graph 910 of figure 17 illustrates first detection signals 912.

Graph 920 of figure 17 illustrates first filtered detection signal 922 that includes first cardiac cycle waveforms 922(1) - 922(N).

Graph 930 of figure 17 illustrates first cardiac cycle waveform quality scores 932 of first cardiac cycle waveforms 922(1) - 922(N).

Graph 940 of figure 17 illustrates first waveform template 950, first duration-normalized cardiac cycle waveforms 960 of a fixed duration 970. The first cardiac cycle waveforms were converted to become the first duration-normalized cardiac cycle waveforms 960.

The x-axis of graphs 910, 920, 930 and 940 represent time while the y-axis of graphs 910, 920 and 940 represent intensity.

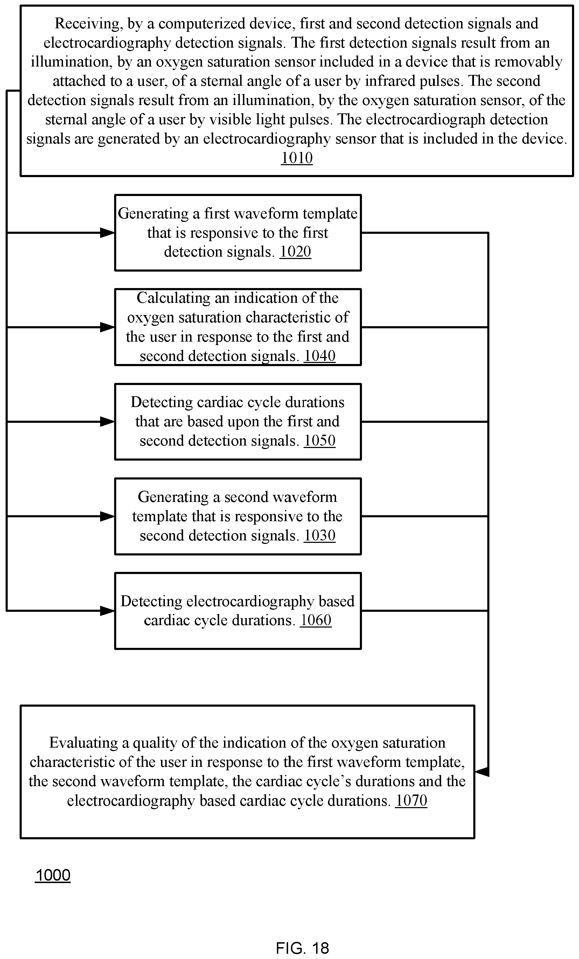

Figure 18 illustrates method 1000 of evaluating a quality of an indication of the oxygen saturation characteristic of the user according to an embodiment of the invention.

Method 1000 may start with stage 1010 of receiving, by a computerized device, first and second detection signals and electrocardiograph signals. The first detection signals result from an illumination, by an oxygen saturation sensor included in a device that is removably attached to a user, of a sternal angle of a user by infrared pulses. The second detection signals result from an illumination, by the oxygen saturation sensor, of the sternal angle of a user by visible light pulses. The electrocardiograph signals are detected by an electrocardiography sensor that is included in the device.

Stage 1010 may be followed by stages 1020, 1030, 1040, 1050 and 1060.

Stage 1020 may include generating a first waveform template that is responsive to the first detection signals.

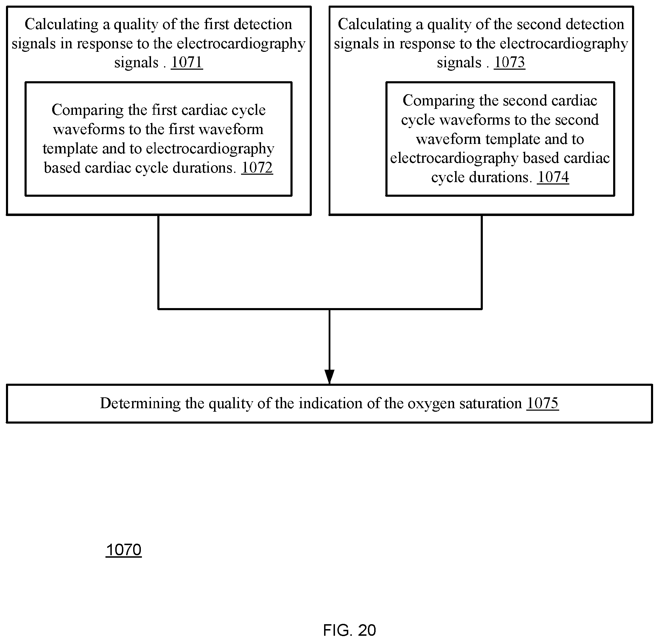

Stage 1020 may include at least one of stages 721-726, 728 and 729 of Figure 13. Further detail of stage 1020 is shown in Figure 20.

Stage 1030 may include generating a second waveform template that is responsive to the second detection signals.

Stage 1030 may include at least one of stages 721'-726', 728' and 729' of figure 14.

Stage 1040 may include calculating an indication of the oxygen saturation characteristic of the user in response to the first and second detection signals.

Stage 1050 may include detecting cardiac cycle durations that are based upon the first and second detection signals.

Stage 1050 may include stages 721, 722, 721' and 722' of Figures 13 and 14.

Stage 1060 may include detecting electrocardiography based cardiac cycle durations.

Stages 1020, 1030, 1040, 1050 and 1060 may be followed by stage 1070 of evaluating a quality of the indication of the oxygen saturation characteristic of the user in response to the first waveform template, the second waveform template, the cardiac cycle's durations and the electrocardiography based cardiac cycle durations.

Stage 1070 may include at least one of stages 730, 731, 732, 733, 734, 735,736,737,730', 731', 732', 733', 734', 735', 736', 737' and 739' of figures 11-13.

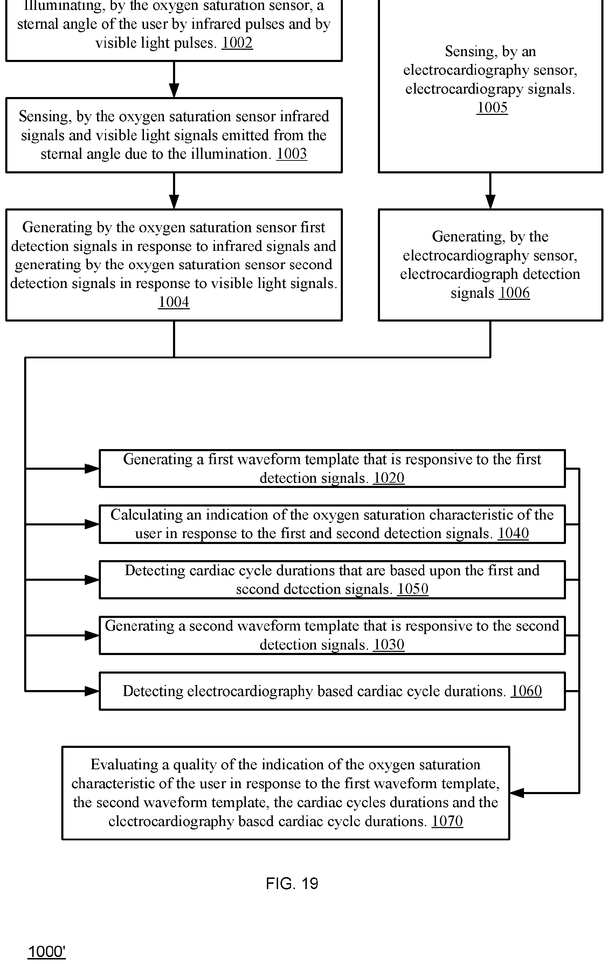

Figure 19 illustrates method 1000' evaluating a quality of an indication of the oxygen saturation characteristic of the user according to an embodiment of the invention.

Method 1000' may start with stages 1002 and 1005.

Stage 1002 may include illuminating, by the oxygen saturation sensor, a sternal angle of the user by infrared pulses and by visible light pulses.

Stage 1002 may be followed by stage 1003 of sensing, by the oxygen saturation sensor infrared signals and visible light signals emitted from the sternal angle due to the illumination.

Stage 1003 may be followed by stage 1004 of generating by the oxygen saturation sensor first detection signals in response to infrared signals and generating by the oxygen saturation sensor second detection signals in response to visible light signals.

Stage 1005 may include sensing, by an electrocardiography sensor, electrocardiography signals.

Stage 1005 may be followed by stage 1006 of generating, by the electrocardiography sensor, electrocardiograph detection signals.

Stages 1004 and 1002 may be executed in parallel to each other, m a partially overlapping manner or in a non-overlapping manner. The method can benefit from sensing the same cardiac cycles by the oxygen saturation sensor and the electrocardiography sensor.

Stage 1004 and stage 1006 may be followed by stages 1020, 1030, 1040, 1050 and 1060.

Stage 1020 may include generating a first waveform template that is responsive to the first detection signals. Further detail of stage 1020 is shown in Figure 20.

Stage 1030 may include generating a second waveform template that is responsive to the second detection signals.

Stage 1040 may include calculating an indication of the oxygen saturation characteristic of the user in response to the first and second detection signals.

Stage 1050 may include detecting cardiac cycle durations that are based upon the first and second detection signals.

Stage 1060 may include detecting electrocardiography based cardiac cycle durations.

Stages 1020, 1030, 1040, 1050 and 1060 may be followed by stage 1070 of evaluating a quality of the indication of the oxygen saturation characteristic of the user in response to the first waveform template, the second waveform template, the cardiac cycle's durations and the electrocardiography based cardiac cycle durations.

Figure 20 illustrates stage 1070 of Figure 18 according to an embodiment of the invention.

Stage 1070 may start by stages 1071 and 1073.

Stage 1071 may include calculating a quality of the first detection signals in response to the electrocardiography signals.

Stage 1071 may include stage 1072 of comparing the first cardiac cycle waveforms to the first waveform template and to electrocardiography based cardiac cycle durations.

Stage 1073 may include calculating a quality of the second detection signals in response to the electrocardiography signals.

Stage 1073 may include stage 1074 may include comparing the second cardiac cycle waveforms to the second waveform template and to electrocardiography based cardiac cycle durations.

Stage 1071 and 1073 may be followed by stage 1075 of determining the quality of the indication of the oxygen saturation. This may include applying any function on the quality of the first detection signals and (b) the quality of the second detection signals.

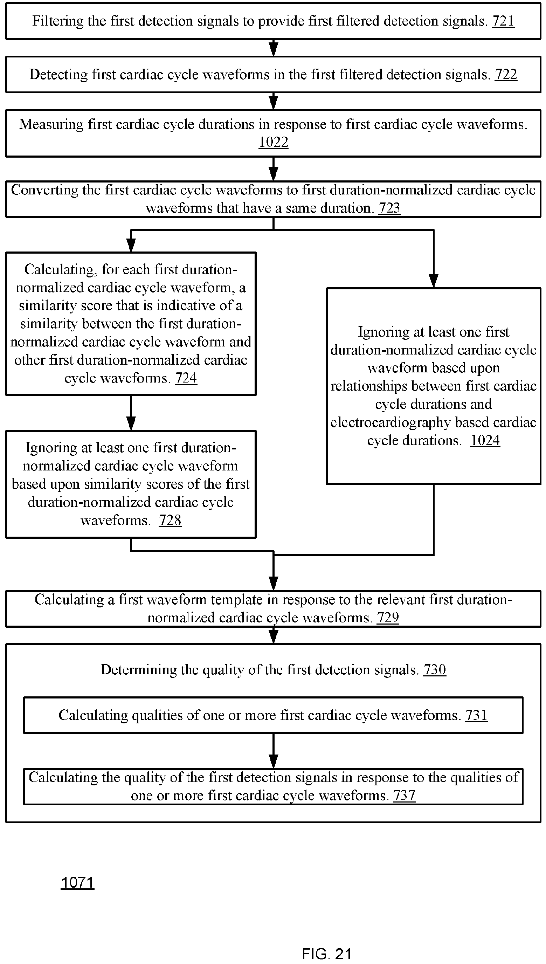

Figure 21 illustrates a stage 1071 for calculating a quality of the first detection signals in response to the electrocardiography signals according to an embodiment of the invention.

Stage 721 may include filtering the first detection signals to provide first filtered detection signals. The filtering may include high-pass filtering and low-pass filtering or applying bandpass filtering. The low-pass filtering may be bilateral filtering, any other edge preserving filtering or any other filtering.

Stage 721 may be followed by stage 722 of detecting first cardiac cycle waveforms in the first filtered detection signals.

Stage 722 may be followed by stage 723 of converting the first cardiac cycle waveforms to first duration-normalized cardiac cycle waveforms that have a same duration.

Stage 723 may be followed by one or more branches. A first branch (also shown in figure 13) includes stages 724 and 728 and a second branch includes stage 1024. Both branches are followed by stage 729.

Stage 1024 may include ignoring at least one first duration-normalized cardiac cycle waveform based upon relationships between first cardiac cycle durations and electrocardiography based cardiac cycle durations.

Stage 724 may include calculating, for each first duration-normalized cardiac cycle waveform, a similarity score that is indicative of a similarity between the first duration-normalized cardiac cycle waveform and other first duration-normalized cardiac cycle waveforms.

Stage 724 may include stages (not shown) such as stages 725 and 726 of figure 13.

Stage 724 may be followed by stage 728 of ignoring at least one first duration-normalized cardiac cycle waveform based upon similarity scores of the first duration-normalized cardiac cycle waveforms. Stage 728 provides relevant first duration- normalized cardiac cycle waveforms (those first duration-normalized cardiac cycle waveform that were not ignored of).

Stage 728 may include, for example, ignoring one or more first duration-normalized cardiac cycle waveform that have a similarity score that is below a similarity score threshold, or ignoring a preset number of first duration-normalized cardiac cycle waveforms that have the lowest similarity scores.

Stage 729 may include calculating a first waveform template in response to the relevant first duration-normalized cardiac cycle waveforms. This stage may include applying a second mathematical function on the relevant first duration-normalized cardiac cycle waveforms. The second mathematical function may be any mathematical function. It may be, for example, a weighted averaging function or an averaging function.

Stage 729 may be followed by stage 730 of determining the quality of the first detection signals.

Stage 730 may include stage 731 of calculating qualities of one or more first cardiac cycle waveforms. These one or more first cardiac cycle waveforms may include all the first cardiac cycle waveforms detected during stage 722 or only some of the first cardiac cycle waveforms detected during stage 722. For example - the one or more first cardiac cycle waveforms may correspond to the relevant first duration- normalized cardiac cycle waveforms.

Stage 731 may include at least one out of stages (not shown in figure 21 but illustrated in figure 13) 732, 733, 734, 735 and 736.

Stage 730 may include stage 737 of calculating the quality of the first detection signals in response to the qualities (calculated during stage 731) of one or more first cardiac cycle waveforms.

According to the invention a method for measuring oxygen saturation of a user is provided. The method may be executed by any medical device (computerized device) illustrated in this patent application. The method may include receiving, by a computerized device that is detachably attached to a user, first detection signals, second detection signals and electrocardiograph signals;

wherein the first and second detection signals are obtained by illuminating a sternum angle of the user.

Either one of the first detection signals, second detection signals and electrocardiograph signals may be obtained by any method illustrated in in this patent application - but may be obtained in one or more other manners. The method also includes evaluating, by the computerized device, a state of the user in response to the first detection signals, the second detection signals and electrocardiograph signals. The state of the user may reflect the oxygen saturation of the user or any physical parameter of the user.

## Claims

1. A method (1000, 1000') for measuring oxygen saturation of a user, the method comprises:

   receiving, by a computerized device, first and second detection signals (912) and electrocardiograph signals;
   wherein the electrocardiograph signals are detected by an electrocardiography sensor (101', 102', 103', 104') that is included in the device;
   calculating an indication of the oxygen saturation characteristic of the user in response to the first and second detection signals (912); and
   detecting cardiac cycle durations that are based upon the first and second detection signals (912);
   **characterized in that**
   the first detection signals (912) result from an illumination, by an oxygen saturation sensor (150) included in a device (100, 100') that is removably attached to a user, of a sternal angle of a user by infrared pulses (314);
   the second detection signals result from an illumination, by the oxygen saturation sensor (150), of the sternal angle of a user by visible light pulses (304);
   a first waveform template (950) that is responsive to the first detection signals (912) is generated;
   a second waveform template that is responsive to the second detection signals is generated;
   electrocardiography based cardiac cycle durations are detected; and
   a quality of the indication of the oxygen saturation characteristic of the user in response to the first waveform template (950), the second waveform template, the cardiac cycle's durations and the electrocardiography based cardiac cycle durations is evaluated.

2. The method (1000, 1000') according to claim 1 comprising applying a high-pass filter and a bilateral filter on the first detection signals (912) to provide first filtered detection signals (894).

3. The method (1000, 1000') according to claim 1 wherein the generating of the first waveform template (950) comprises filtering the first detection signals (912) to provide first filtered detection signals (922);

and detecting first cardiac cycle waveforms (922(1)-922(N)) in the first filtered detection signals (922), wherein the detecting of the cardiac cycle durations preferably comprises measuring durations of the first cardiac cycle waveforms (922(1)- 922(N)).

4. The method (1000, 1000') according to claim 3 wherein the generating of the first waveform template (950) comprises converting the first cardiac cycle waveforms (922(1)-922(N)) to first duration-normalized cardiac cycle waveforms (960) that have a same duration,

wherein the generating of the first waveform template (950) is preferably responsive to at least some of the first duration-normalized cardiac cycle waveforms (960), or

wherein the generating of the first waveform template (950) preferably equals an average of at least some of the first duration-normalized cardiac cycle waveforms (960).

5. The method (1000, 1000') according to claim 4 wherein the generating of the first waveform template (950) further comprises calculating, for each first duration-normalized cardiac cycle waveform (960), a similarity score indicative of a similarity between the first duration-normalized cardiac cycle waveform (960) and other first duration-normalized cardiac cycle waveforms (960),

wherein the generating of the first waveform template (950) preferably further comprises ignoring at least one first duration-normalized cardiac cycle waveform (960) based upon similarity scores of the first duration-normalized cardiac cycle waveforms (960), or

wherein the calculating, for each first duration-normalized cardiac cycle waveform (960), of the similarity score preferably comprises calculating a plurality of Pearson correlation coefficients between the first duration-normalized cardiac cycle waveform (960) and a plurality of other first duration-normalized cardiac cycle waveforms (960)

wherein the calculating, for each first duration-normalized cardiac cycle waveform (960), of the similarity score more preferably comprises summing the plurality of Pearson correlation coefficients.

6. The method (1000, 1000') according to claim 3 comprising calculating qualities of at least some of the first cardiac cycle waveforms (922(1)- 922(N)); and wherein the quality of the first and second detection signals (912) is responsive to the qualities of the at least some of the first cardiac cycle waveform (922(1)- 922(N)),

wherein the calculating of the qualities of at least some of the first cardiac cycle waveforms (922(1)- 922(N)) preferably comprises comparing the at least some of the first cardiac cycle waveforms (922(1)- 922(N)) to the first waveform template (950), or

wherein the calculating of the qualities of at least some of the first cardiac cycle waveforms (922(1)- 922(N)) preferably comprises calculating correlations between shapes of the at least some of the first cardiac cycle waveforms (922(1)- 922(N)) and a shape of the first waveform template (950), or

wherein the calculating of the qualities of at least some of the first cardiac cycle waveforms (922(1)- 922(N)) preferably comprises converting at least some of the first cardiac cycle waveforms (922(1)- 922(N)) to first duration-normalized and peak-normalized cardiac cycle waveforms and calculating relationships between shapes of the first duration-normalized and peak-normalized cardiac cycle waveforms and a shape of the first waveform template (950); and

wherein the first duration-normalized and peak-normalized cardiac cycle waveforms are a same duration and a same peak value as the first waveform template (950), or

wherein the calculating of the qualities of at least some of the first cardiac cycle waveforms (922(1)- 922(N)) preferably comprises calculating relationships between peaks of the at least some of the first cardiac cycle waveforms (922(1)- 922(N)) and a peak of the first waveform template (950), or

wherein the calculating of the qualities of at least some of the first cardiac cycle waveforms (922(1)- 922(N)) preferably comprises calculating relationships between durations of the at least some of the first cardiac cycle waveforms (922(1)- 922(N)) and durations of corresponding electrocardiography based cardiac cycle durations.

7. The method (1000, 1000') of claim 1, wherein evaluating a quality of the indication of the oxygen saturation characteristic of the user comprises determining, based on the first waveform template (950), the second waveform template, the cardiac cycle's durations and the electrocardiography based cardiac cycle durations, a position of the oxygen saturation sensor (150).

8. The method (1000, 1000') of claim 1, wherein the oxygen saturation sensor (150) is included in the computerized device.

9. The method (1000, 1000') of claim 1, wherein the receiving of the first and second detection signals (912) comprises receiving the first and second detection signals (912) from a different device than the computerized device.

10. The method (1000, 1000') of claim 1, further comprising determining that the detected sternal angle of the user is an actual sternal angle of the user when the quality of the first and second detection signals (912) exceeds a predetermined quality threshold.

**Patentansprüche**

1. Ein Verfahren (1000, 1000') zum Messen der Sauerstoffsättigung eines Benutzers, wobei das Verfahren umfasst:

Empfangen von ersten und zweiten Detektionssignalen (912) und Elektrokardiographie-Signalen durch eine computerisierte Vorrichtung; wobei die Elektrokardiographie-Signale von einem Elektrokardiographie-Sensor (101', 102', 103', 104') erfasst werden, der in der Vorrichtung enthalten ist; Berechnen einer Anzeige der Sauerstoffsättigungscharakteristik des Benutzers in Reaktion auf die ersten und zweiten Erfassungssignale (912); und Erfassen von Herzzyklusdauern, die auf den ersten und zweiten Erfassungssignalen (912) beruhen; **dadurch gekennzeichnet, dass** die ersten Erfassungssignale (912) aus einer Bestrahlung eines Sternumwinkels eines Benutzers durch einen Sauerstoffsättigungssensor (150) mittels Infrarot-Pulsen (314) resultieren, der in einer Vorrichtung (100, 100') enthalten ist, die entfernbar an einem Benutzer angebracht ist; die zweiten Erfassungssignale aus einer Bestrahlung des Sternumwinkels eines Benutzers durch sichtbare Lichtpulse (304) durch den Sauerstoffsättigungssensor (150) resultieren; ein erstes Wellenformmuster (950), das auf die ersten Erfassungssignale (912) anspricht, erzeugt wird; ein zweites Wellenformmuster, das auf die zweiten Erfassungssignale anspricht, erzeugt wird; Elektrokardiographie-basierte Herzzyklusdauern erfasst werden; und

eine Qualität der Anzeige der Sauerstoffsättigungscharakteristik des Benutzers in Reaktion auf das erste Wellenformmuster (950), das zweite Wellenformmuster, die Herzzyklusdauern und die Elektrokardiographie-basierten Herzzyklusdauern ausgewertet wird.

2. Verfahren (1000, 1000') nach Anspruch 1, umfassend das Anwenden eines Hochpassfilters und eines bilateralen Filters auf die ersten Erfassungssignale (912), um erste gefilterte Erfassungssignale (894) bereitzustellen.

3. Verfahren (1000, 1000') nach Anspruch 1, wobei das Erzeugen des ersten Wellenformmusters (950) das Filtern der ersten Erfassungssignale (912) umfasst, um erste gefilterte Erfassungssignale (922) bereitzustellen;

und das Erfassen erster Herzzyklus-Wellenformen (922(1)-922(N)) in den ersten gefilterten Erfassungssignalen (922), wobei das Erfassen der Herzzyklusdauern vorzugsweise das Messen von Dauern der ersten Herzzykluswellenformen (922(1)-922(N)) umfasst.

4. Verfahren (1000, 1000') nach Anspruch 3, wobei das Erzeugen des ersten Wellenformmusters (950) das Umwandeln der ersten Herzzyklus-Wellenformen (922(1)-922(N)) in erste Dauer-normalisierte Herzzyklus-Wellenformen (960) umfasst, die eine gleiche Dauer aufweisen,

wobei das Erzeugen des ersten Wellenformmusters (950) vorzugsweise auf mindestens einige der ersten Dauer-normalisierten Herzzyklus-Wellenformen (960) anspricht, oder wobei das Erzeugen des ersten Wellenformmusters (950) vorzugsweise einem Mittelwert von zumindest einigen der ersten Dauer-normalisierten Herzzyklus-Wellenformen (960) entspricht.

5. Verfahren (1000, 1000') nach Anspruch 4, wobei das Erzeugen des ersten Wellenformmusters (950) ferner das Berechnen eines Ähnlichkeitsscores, der eine Ähnlichkeit zwischen der ersten Dauer-normalisierten Herzzyklus-Wellenform (960) und anderen ersten Dauer-normalisierten Herzzyklus-Wellenformen (960) anzeigt, für jede erste Dauer-normalisierte Herzzyklus-Wellenform (960) umfasst,

wobei das Erzeugen des ersten Wellenformmusters (950) vorzugsweise weiterhin das Ignorieren mindestens einer ersten Dauer-normalisierten Herzzyklus-Wellenform (960) auf der Grundlage von Ähnlichkeitsscores der ersten Dauer-normalisierten Herzzyklus-Wellenformen (960) umfasst, oder

wobei das Berechnen des Ähnlichkeitsscores für jede erste Dauer-normalisierte Herzzyklus-Wellenform (960) vorzugsweise das Berechnen einer Vielzahl von Pearson-Korrelationskoeffizienten zwischen der ersten Dauer-normalisierten Herzzyklus-Wellenform (960) und einer Vielzahl von anderen ersten Dauer-normalisierten Herzzyklus-Wellenformen (960) umfasst wobei das Berechnen der Ähnlichkeitsscores für jede erste Dauer-normalisierte Herzzyklus-Wellenform (960) vorzugsweise das Summieren der Mehrzahl von Pearson-Korrelationskoeffizienten umfasst.

6. Verfahren (1000, 1000') nach Anspruch 3, umfassend das Berechnen von Qualitäten von zumindest einigen der ersten Herzzyklus-Wellenformen (922(1)- 922(N)); und wobei die Qualität der ersten und zweiten Erfassungssignale (912) auf die Qualitäten der zumindest einigen der ersten Herzzyklus-Wellenform (922(1)- 922(N)) anspricht,

wobei das Berechnen der Qualitäten von zumindest einigen der ersten Herzzykluswellenformen (922(1)-922(N)) vorzugsweise das Vergleichen der zumindest einigen der ersten Herzzykluswellenformen (922(1)-922(N)) mit der ersten Wellenformschablone (950) umfasst, oder wobei das Berechnen der Qualitäten von zumindest einigen der ersten Herzzyklus-Wellenformen (922(1)- 922(N)) vorzugsweise das Berechnen von Korrelationen zwischen Formen von zumindest einigen der ersten Herzzyklus-Wellenformen (922(1)- 922(N)) und einer Form des ersten Wellenformmusters (950) umfasst, oder wobei das Berechnen der Qualitäten von zumindest einigen der ersten Herzzyklus-Wellenformen (922(1)-922(N)) vorzugsweise das Umwandeln von zumindest einigen der ersten Herzzyklus-Wellenformen (922(1)-922(N)) in erste Dauernormalisierte und Spitzenwert-normalisierte Herzzyklus-Wellenformen und das Berechnen von Beziehungen zwischen Formen der ersten Dauernormalisierten und Spitzenwert-normalisierten Herzzyklus-Wellenformen und einer Form des ersten Wellenformmusters (950) umfasst; und wobei die ersten Dauer-normalisierten und Spitzenwert-normalisierten Herzzyklus-Wellenformen eine gleiche Dauer und einen gleichen Spitzenwert wie das erste Wellenformmuster (950) aufweisen, oder wobei das Berechnen der Qualitäten von zumindest einigen der ersten Herzzyklus-Wellenformen (922(1)-922(N)) vorzugsweise das Berechnen von Beziehungen zwischen Spitzenwerten von zumindest einigen der ersten Herzzyklus-Wellenformen (922(1)-922(N)) und einem Spitzenwert des ersten Wellenformmusters (950) umfasst, oder wobei das Berechnen der Qualitäten von zumindest einigen der ersten Herzzyklus-Wellenformen (922(1)-922(N)) vorzugsweise das Berechnen von Beziehungen zwischen Dauern von zumindest einigen der ersten Herzzyklus-Wellenformen (922(1)-922(N)) und Dauern von entsprechenden Elektrokardiographie-basierten Herzzyklus-Dauern umfasst.

7. Verfahren (1000, 1000') nach Anspruch 1, wobei das Auswerten einer Qualität der Anzeige der Sauerstoffsättigungscharakteristik des Benutzers das Bestimmen einer Position des Sauerstoffsättigungssensors (150) auf der Grundlage des ersten Wellenformmusters (950), der zweiten Wellenformschablone, der Herzzyklusdauern und der Elektrokardiographie-basierten Herzzyklusdauern umfasst.

8. Verfahren (1000, 1000') nach Anspruch 1, wobei der Sauerstoffsättigungssensor (150) in der computerisierten Vorrichtung enthalten ist.

9. Verfahren (1000, 1000') nach Anspruch 1, wobei das Empfangen der ersten und zweiten Erfassungssignale (912) das Empfangen der ersten und zweiten Erfassungssignale (912) von einer anderen Vorrichtung als der computerisierten Vorrichtung umfasst.

10. Verfahren (1000, 1000') nach Anspruch 1, ferner umfassend das Bestimmen, dass der detektierte Sternum-Winkel des Benutzers ein tatsächlicher Sternum-Winkel des Benutzers ist, wenn die Qualität der ersten und zweiten Detektionssignale (912) eine vorbestimmte Qualitätsschwelle überschreitet.

**Revendications**

1. Procédé (1000, 1000') pour mesurer une saturation en oxygène d'un utilisateur, le procédé comprend :

la réception, par un dispositif informatisé, de premiers et de seconds signaux de détection (912) et signaux électrocardiographes ; dans lequel les signaux électrocardiographes sont détectés par un capteur d'électrocardiographie (101', 102', 103', 104') qui est inclus dans le dispositif ; le calcul d'une indication de la caractéristique de saturation en oxygène de l'utilisateur en réponse aux premiers et aux seconds signaux de détection (912) ; et la détection de durées de cycle cardiaque qui sont sur la base des premiers et des seconds signaux de détection (912) ;

**caractérisé en ce que**

les premiers signaux de détection (912) résultent d'une illumination, par un capteur de saturation en oxygène (150) inclus dans un dispositif (100, 100') qui est attaché de manière amovible à un utilisateur, d'un angle sternal d'un utilisateur par des impulsions infrarouges (314) ;

les seconds signaux de détection résultent d'une illumination, par le capteur de saturation en oxygène (150), de l'angle sternal d'un utilisateur par des impulsions de lumière visible (304) ;

un premier modèle de forme d'onde (950) qui est en réponse aux premiers signaux de détection (912) est généré ;

un second modèle de forme d'onde qui est en réponse aux seconds signaux de détection est généré ;

des durées de cycle cardiaque sur la base d'électrocardiographie sont détectées ; et

une qualité de l'indication de la caractéristique de la saturation en oxygène de l'utilisateur en réponse au premier modèle de forme d'onde (950), au second modèle de forme d'onde, aux durées de cycle cardiaque et aux durées de cycle cardiaque sur la base d'électrocardiographie est évaluée.

2. Procédé (1000, 1000') selon la revendication 1 comprenant le fait d'appliquer un filtre passe-haut et un filtre bilatéral sur les premiers signaux de détection (912) pour fournir des premiers signaux de détection filtrés (894).

3. Procédé (1000, 1000') selon la revendication 1 dans lequel la génération du premier modèle de forme d'onde (950) comprend le filtrage des premiers signaux de détection (912) pour fournir des premiers signaux de détection filtrés (922) ;

et la détection de premières formes d'onde de cycle cardiaque (922(1)-922(N)) dans les premiers signaux de détection filtrés (922), dans lequel la détection des durées de cycle cardiaque comprend préférablement la mesure de durées des premières formes d'onde de cycle cardiaque (922(1)-922(N)).

4. Procédé (1000, 1000') selon la revendication 3 dans lequel la génération du premier modèle de forme d'onde (950) comprend la conversion des premières formes d'onde de cycle cardiaque (922(1)-922(N)) en premières formes d'onde de cycle cardiaque normalisées en durée (960) qui ont une même durée, dans lequel la génération du premier modèle de forme d'onde (950) est préférablement en réponse à au moins une partie des premières formes d'onde de cycle cardiaque normalisées en durée (960), ou dans lequel la génération du premier modèle de forme d'onde (950) est égale préférablement à une moyenne d'au moins une partie des premières formes d'onde de cycle cardiaque normalisées en durée (960).

5. Procédé (1000, 1000') selon la revendication 4 dans lequel la génération du premier modèle de forme d'onde (950) comprend en outre le calcul, pour chaque première forme d'onde de cycle cardiaque normalisée en durée (960), d'un indice de similarité indicatif d'une similarité entre la première forme d'onde de cycle cardiaque normalisée en durée (960) et d'autres premières formes d'onde de cycle cardiaque normalisées en durée (960),

dans lequel la génération du premier modèle de forme d'onde (950) comprend en outre préférablement le fait d'ignorer au moins une première forme d'onde de cycle cardiaque normalisée en durée (960) sur la base d'indices de similarité des premières formes d'onde de cycle cardiaque normalisées en durée (960), ou dans lequel le calcul, pour chaque première forme d'onde de cycle cardiaque normalisée en durée (960), de l'indice de similarité comprend préférablement le calcul d'une pluralité de coefficients de corrélation de Pearson entre la première forme d'onde de cycle cardiaque normalisée en durée (960) et une pluralité d'autres premières formes d'onde de cycle cardiaque normalisées en durée (960) dans lequel le calcul, pour chaque première forme d'onde de cycle cardiaque normalisée en durée (960), de l'indice de similarité comprend plus préférablement le fait de sommer la pluralité de coefficients de corrélation de Pearson.

6. Procédé (1000, 1000') selon la revendication 3 comprenant le calcul de qualités d'au moins une partie des premières formes d'onde de cycle cardiaque (922(1)-922(N)) ; et dans lequel la qualité des premiers et des seconds signaux de détection (912) est en réponse aux qualités de l'au moins une partie de la première forme d'onde de cycle cardiaque (922(1)-922(N)),

dans lequel le calcul des qualités d'au moins une partie des premières formes d'onde de cycle cardiaque (922(1)-922(N)) comprend préférablement la comparaison de l'au moins une partie des premières formes d'onde de cycle cardiaque (922(1)-922(N)) au premier modèle de forme d'onde (950), ou dans lequel le calcul des qualités d'au moins une partie des premières formes d'onde de cycle cardiaque (922(1)-922(N)) comprend préférablement le calcul de corrélations entre des formes de l'au moins une partie des premières formes d'onde de cycle cardiaque (922(1)-922(N)) et une forme du premier modèle de forme d'onde (950), ou

dans lequel le calcul des qualités d'au moins une partie des premières formes d'onde de cycle cardiaque (922(1)-922(N)) comprend préférablement la conversion d'au moins une partie des premières formes d'onde de cycle cardiaque (922(1)-922(N)) aux premières formes d'onde de cycle cardiaque normalisées en durée et normalisées en pic et le calcul de relations entre des formes des premières formes d'onde de cycle cardiaque normalisées en durée et normalisées en pic et une forme du premier modèle de forme d'onde (950) ; et

dans lequel les premières formes d'onde de cycle cardiaque normalisées en durée et normalisées en pic sont d'une même durée et d'une même valeur pic que le premier modèle de forme d'onde (950), ou

dans lequel le calcul des qualités d'au moins une partie des premières formes d'onde de cycle cardiaque (922(1)-922(N)) comprend préférablement le calcul de relations entre des pics de l'au moins une partie des premières formes d'onde de cycle cardiaque (922(1)-922(N)) et un pic du premier modèle de forme d'onde (950), ou

dans lequel le calcul des qualités d'au moins une partie des premières formes d'onde de cycle cardiaque (922(1)-922(N)) comprend préférablement le calcul de relations entre des durées de l'au moins une partie des premières formes d'onde de cycle cardiaque (922(1)-922(N)) et des durées de durées de cycle cardiaque sur la base d'électrocardiographie correspondant.

7. Procédé (1000, 1000') selon la revendication 1, dans lequel l'évaluation d'une qualité de l'indication de la caractéristique de la saturation en oxygène de l'utilisateur comprend la détermination, sur la base du premier modèle de forme d'onde (950), du second modèle de forme d'onde, des durées de cycle cardiaque et des durées de cycle cardiaque sur la base d'électrocardiographie, d'une position du capteur de saturation en oxygène (150).

8. Procédé (1000, 1000') selon la revendication 1, dans lequel le capteur de saturation en oxygène (150) est inclus dans le dispositif informatisé.

9. Procédé (1000, 1000') selon la revendication 1, dans lequel la réception des premiers et des seconds signaux de détection (912) comprend la réception des premiers et des seconds signaux de détection (912) d'un dispositif différent du dispositif informatisé.

10. Procédé (1000, 1000') selon la revendication 1, comprenant en outre la détermination que l'angle sternal détecté de l'utilisateur est un vrai angle sternal de l'utilisateur lorsque la qualité des premiers et des seconds signaux de détection (912) dépasse un seuil de qualité prédéterminé.

FIG. 1

FIG. 2

FIG. 3

FIG. 4

FIG. 5

EP 3 288 457 B1

RED
diode
ON

Data
sampling
303

RED
diode
OFF

IR
diode
ON

Data
sampling
313

IR
diode
OFF

IDLE 333

Illumination
sequence

301

311

Light stabilization period

302

312

304

314

330

300

FIG. 6

Attaching a device that includes a oxygen saturation sensor so that the oxygen saturation sensor faces the sternal angle. 410

Performing oxygen saturation measurements. 420

Illuminating a sternal angle of the user by electromagnetic radiation. 422

Sensing, by a oxygen saturation sensor included in a device that is removably attached to a user, radiation emitted from the sternal angle of the user as a result of the illuminating, wherein the sensing occurs while the oxygen saturation sensor faces the sternal angle of the user. 424

Generating detection signals by the oxygen saturation sensor in response to the sensing of the radiation, wherein the detection signals are indicative of an oxygen saturation characteristic of the user. 426

Processing the detection signals generated by the oxygen saturation sensor to provide an indication of the oxygen saturation characteristic of the user. 428

Wirelessly transmitting by a transmitter of the device information about the oxygen saturation characteristic of the user. 430

Feeding the processor and the oxygen saturation sensor with power from a battery. The battery may be positioned within a lower case of the device; wherein the processor is positioned within an upper case of the device. 480

400

FIG. 7

Attaching a device that includes a oxygen saturation sensor so that the oxygen saturation sensor faces the sternal angle. 510

Sensing, by a movement sensor of the device, a movement of the user during the sensing of the radiation. 520

Performing oxygen saturation measurements. 550

Illuminating a sternal angle of the user by electromagnetic radiation. 422

Sensing, by a oxygen saturation sensor included in a device that is removably attached to a user, radiation emitted from the sternal angle of the user as a result of the illuminating, wherein the sensing occurs while the oxygen saturation sensor faces the sternal angle of the user. 424

Determining an accuracy of the detection signals in response to movement of the user. 530

Generating detection signals by the oxygen saturation sensor in response to the sensing of the radiation, wherein the detection signals are indicative of an oxygen saturation characteristic of the user. 426

Processing the detection signals generated by the oxygen saturation sensor to provide an indication of the oxygen saturation characteristic of the user. 552

Rejecting detection signals that represent radiation sensed when the user movement exceeds a movement threshold. 554

Feeding the processor and the oxygen saturation sensor with power from a battery. 580

Sensing a temperature of the user. 570

Wirelessly transmitting by a transmitter of the device information about the oxygen saturation characteristic of the user. 530

500

FIG. 8

Attaching a device that includes an oxygen saturation sensor so that the oxygen saturation sensor faces the sternal angle. 510

Performing oxygen saturation measurements. 550

Illuminating a sternal angle of the user by electromagnetic radiation. 422

Sensing, by a oxygen saturation sensor included in a device that is removably attached to a user, radiation emitted from the sternal angle of the user. The radiation can be emitted as a result of the illuminating, from ambient radiaiton or a combination of both. The sensing occurs while the oxygen saturation sensor faces the sternal angle of the user. 624

Generating detection signals by the oxygen saturation sensor in response to the sensing of the radiation, wherein the detection signals may be indicative of an oxygen saturation characteristic of the user. 426

Processing the detection signals by the oxygen saturation sensor to provide an indication of the oxygen saturation characteristic of the user and detecting ambient illumination of the sternal angle by processing detection signals generated in response to sensing radiation emitted from the sternal angle at points in time where the sternal angle is not illuminated by the illumination module of the device. 628

Responding to the detection of ambient illumination. For example, calibrating device or generating an alert indicative of a detection of the ambient illumination. 629

Feeding the processor and the oxygen saturation sensor with power from a battery. 580

Wirelessly transmitting by a transmitter of the device information about the oxygen saturation characteristic of the user. 530

600

FIG. 9

29

FIG. 10

Positioning the device so that the oxygen saturation sensor of the device illuminates the sternal angle or illuminate an area that is proximate to the sternal angle. 710

Illuminating, by the oxygen saturation sensor, the current portion of the sternum of the user by infrared pulses and by visible light pulses. 712

Sensing, by the oxygen saturation sensor infrared signals and visible light signals emitted from the current portion of the sternum due to the illumination of the current portion of the sternum by the infrared pulses and the visible light pulses respectively. 714

Generating first and second detection signals, by the oxygen saturation sensor, in response to the sensing of the infrared signals and visible light signals. The first and second detection signals are indicative of an oxygen saturation characteristic of the user. 716

Processing the first and second detection signals to evaluate a combined quality of the first and second detection signals. 720

Determining whether the current portion of the sternum of the user is the sternal angle of the user; wherein the determining is responsive to the combined quality of the first and second detection signals. 740

failure | failure | Success

Declaring a positioning failure and ending the positioning process. 770

Selecting a new current portion of the sternum to be illuminated, instructing the user to move the device so that the oxygen saturation sensor illuminates the new current portion 760

Generating a positioning success indication. 750

700

FIG. 11

Receiving, by a computerized device, first detection signals generated as a result of an illumination, by infrared pulses, of a first portion of a sternum of a user. Receiving, by the computerized device, second detection signals generated as a result of an illumination, by visible light pulses, of the first portion of the sternum of the user. 810

Calculating an oxygen saturation of the user, based upon the first and second detection signals. 850

Processing the first and second detection signals to evaluate a combined quality of the first and second detection signals. 720

Determining whether the current portion of the sternum of the user is the sternal angle of the user. The determining is responsive to the combined quality of the first and second detection signals. 740

failure

failure

Success

Declaring a positioning failure and ending the positioning process. 770

Selecting a new current portion of the sternum to be illuminated, instructing the user to move the device so that the oxygen saturation sensor illuminates the new current portion 760

Generating a positioning success indication. 750

800

FIG. 12

Filtering the first detection signals to provide first filtered detection signals. 721

↓

Detecting first cardiac cycle waveforms in the first filtered detection signals. 722

↓

Converting the first cardiac cycle waveforms to first duration-normalized cardiac cycle waveforms that have a same duration. 723

↓

Calculating, for each first duration-normalized cardiac cycle waveform, a similarity score that is indicative of a similarity between the first duration-normalized cardiac cycle waveform and other first duration-normalized cardiac cycle waveforms. 724

Calculating, for each first duration normalized cardiac cycle waveform, a plurality of Pearson correlation coefficients between the first duration-normalized cardiac cycle waveform and a plurality of other first duration-normalized cardiac cycle waveforms. 725

↓

Applying a first mathematical function on the plurality of Pearson correlation coefficients to provide the similarity score. 726

↓

Ignoring at least one first duration-normalized cardiac cycle waveform based upon similarity scores of the first duration-normalized cardiac cycle waveforms. 728

↓

Calculating a first waveform template in response to the relevant first duration-normalized cardiac cycle waveforms. 729

↓

Determining the quality of the first detection signals. 730

Calculating qualities of one or more first cardiac cycle waveforms. 731

↓

Calculating the quality of the first detection signals in response to the qualities of one or more first cardiac cycle waveforms. 737

From stage 730'

↓

Calculating a combined quality of the first and second detection signals in response to quality of the first detection signals and to the quality of the second detection signals. 739

720

FIG. 13

Filtering the second detection signals to provide second filtered detection signals. 721'

Detecting second cardiac cycle waveforms in the second filtered detection signals. 722'

Converting the second cardiac cycle waveforms to second duration-normalized cardiac cycle waveforms that have a same duration. 723'

Calculating, for each second duration-normalized cardiac cycle waveform, a similarity score that is indicative of a similarity between the second duration-normalized cardiac cycle waveform and other second duration-normalized cardiac cycle waveforms. 724'

Calculating, for each second duration normalized cardiac cycle waveform, a plurality of Pearson correlation coefficients between the second duration-normalized cardiac cycle waveform and a plurality of other second duration-normalized cardiac cycle waveforms. 725'

Applying a second mathematical function on the plurality of Pearson correlation coefficients to provide the similarity score. 726'

Ignoring at least one second duration-normalized cardiac cycle waveform based upon similarity scores of the second duration-normalized cardiac cycle waveforms. 728'

Calculating a second waveform template in response to the relevant second duration-normalized cardiac cycle waveforms. 729'

Determining the quality of the second detection signals. 730'

Calculating qualities of one or more second cardiac cycle waveforms. 731'

Calculating the quality of the second detection signals in response to the qualities of one or more second cardiac cycle waveforms. 737'

From stage 730

Calculating a combined quality of the second and second detection signals in response to quality of the second detection signals and to the quality of the second detection signals. 739

720

FIG. 14

Comparing the first cardiac cycle waveforms to the first waveform template. 732

Calculating correlations between shapes of the at least some of the first cardiac cycle waveforms and a shape of the first waveform template. 733

Converting at least some of the first cardiac cycle waveforms to first duration-normalized and peak-normalized cardiac cycle waveforms and calculating relationships between shapes of the first duration-normalized and peak-normalized cardiac cycle waveforms and a shape of the first waveform template. 734

Calculating relationships between peaks of the at least some of the first cardiac cycle waveforms and a peak of the first waveform template. 735

Calculating relationships between durations of the at least some of the first cardiac cycle waveforms and a duration of the first waveform template . 736

731

Comparing the second cardiac cycle waveforms to the second waveform template. 732'

Calculating correlations between shapes of the at least some of the second cardiac cycle waveforms and a shape of the second waveform template. 733'

Converting at least some of the second cardiac cycle waveforms to second duration-normalized and peak-normalized cardiac cycle waveforms and calculating relationships between shapes of the second duration-normalized and peak-normalized cardiac cycle waveforms and a shape of the second waveform template. 734'

Calculating relationships between peaks of the at least some of the second cardiac cycle waveforms and a peak of the second waveform template. 735'

Calculating relationships between durations of the at least some of the second cardiac cycle waveforms and a duration of the second waveform template. 736'

731'

FIG. 15

FIG. 16

FIG. 17

Receiving, by a computerized device, first and second detection signals and electrocardiography detection signals. The first detection signals result from an illumination, by an oxygen saturation sensor included in a device that is removably attached to a user, of a sternal angle of a user by infrared pulses. The second detection signals result from an illumination, by the oxygen saturation sensor, of the sternal angle of a user by visible light pulses. The electrocardiograph detection signals are generated by an electrocardiography sensor that is included in the device. 1010

Generating a first waveform template that is responsive to the first detection signals. 1020

Calculating an indication of the oxygen saturation characteristic of the user in response to the first and second detection signals. 1040

Detecting cardiac cycle durations that are based upon the first and second detection signals. 1050

Generating a second waveform template that is responsive to the second detection signals. 1030

Detecting electrocardiography based cardiac cycle durations. 1060

Evaluating a quality of the indication of the oxygen saturation characteristic of the user in response to the first waveform template, the second waveform template, the cardiac cycle's durations and the electrocardiography based cardiac cycle durations. 1070

1000

FIG. 18

Illuminating, by the oxygen saturation sensor, a sternal angle of the user by infrared pulses and by visible light pulses. 1002

Sensing, by an electrocardiography sensor, electrocardiograpy signals. 1005

Sensing, by the oxygen saturation sensor infrared signals and visible light signals emitted from the sternal angle due to the illumination. 1003

Generating by the oxygen saturation sensor first detection signals in response to infrared signals and generating by the oxygen saturation sensor second detection signals in response to visible light signals. 1004

Generating, by the electrocardiography sensor, electrocardiograph detection signals 1006

Generating a first waveform template that is responsive to the first detection signals. 1020

Calculating an indication of the oxygen saturation characteristic of the user in response to the first and second detection signals. 1040

Detecting cardiac cycle durations that are based upon the first and second detection signals. 1050

Generating a second waveform template that is responsive to the second detection signals. 1030

Detecting electrocardiography based cardiac cycle durations. 1060

Evaluating a quality of the indication of the oxygen saturation characteristic of the user in response to the first waveform template, the second waveform template, the cardiac cycles durations and the electrocardiography based cardiac cycle durations. 1070

FIG. 19

1000'

Calculating a quality of the first detection signals in response to the electrocardiography signals . 1071

Comparing the first cardiac cycle waveforms to the first waveform template and to electrocardiography based cardiac cycle durations. 1072

Calculating a quality of the second detection signals in response to the electrocardiography signals . 1073

Comparing the second cardiac cycle waveforms to the second waveform template and to electrocardiography based cardiac cycle durations. 1074

Determining the quality of the indication of the oxygen saturation 1075

1070

FIG. 20

Filtering the first detection signals to provide first filtered detection signals. 721

Detecting first cardiac cycle waveforms in the first filtered detection signals. 722

Measuring first cardiac cycle durations in response to first cardiac cycle waveforms. 1022

Converting the first cardiac cycle waveforms to first duration-normalized cardiac cycle waveforms that have a same duration. 723

Calculating, for each first duration-normalized cardiac cycle waveform, a similarity score that is indicative of a similarity between the first duration-normalized cardiac cycle waveform and other first duration-normalized cardiac cycle waveforms. 724

Ignoring at least one first duration-normalized cardiac cycle waveform based upon relationships between first cardiac cycle durations and electrocardiography based cardiac cycle durations. 1024

Ignoring at least one first duration-normalized cardiac cycle waveform based upon similarity scores of the first duration-normalized cardiac cycle waveforms. 728

Calculating a first waveform template in response to the relevant first duration-normalized cardiac cycle waveforms. 729

Determining the quality of the first detection signals. 730

Calculating qualities of one or more first cardiac cycle waveforms. 731

Calculating the quality of the first detection signals in response to the qualities of one or more first cardiac cycle waveforms. 737

1071

FIG. 21

**EP 3 288 457 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013030259 A1
- WO 2008120154 A2
- US 2004215244 A1
- WO 9843071 A1
- US 2004138538 A1
- WO 2013036718 A1